Europäisches Patentamt

European Patent Office

Office européen des brevets

(19)

(11) **EP 0 665 955 B1**

(12) **EUROPEAN PATENT SPECIFICATION**

(45) Date of publication and mention
of the grant of the patent:
**29.05.2002 Bulletin 2002/22**

(51) Int Cl.[7]: **G01N 33/545**, G01N 33/547,
G01N 33/553, G01N 33/543,
G01N 33/548

(21) Application number: **93923887.9**

(22) Date of filing: **14.10.1993**

(86) International application number:
**PCT/US93/09840**

(87) International publication number:
**WO 94/09368 (28.04.1994 Gazette 1994/10)**

(54) **PARTICLES HAVING GELATIN-AMINODEXTRAN COATINGS OF AND PROCESSES FOR MAKING SAME**

TEILCHEN MIT GELATINE-AMINODEXTRAN-UMHÜLLUNGEN UND VERFAHREN ZU IHRER HERSTELLUNG

PARTICULES COMPRENANT DES ENROBAGES DE GELATINE-AMINODEXTRANE ET LEURS PROCEDES DE PRODUCTION

(84) Designated Contracting States:
**DE FR GB**

(30) Priority: **15.10.1992 US 961157**
**29.10.1992 US 968158**

(43) Date of publication of application:
**09.08.1995 Bulletin 1995/32**

(60) Divisional application:
**01126422.3**

(73) Proprietor: **Coulter International Corporation**
**Miami, Florida 33196 (US)**

(72) Inventors:
• **SIIMAN, Olavi**
**Davie, FL 33331 (US)**
• **BURSHTEYN, Alexander**
**Hialeah, FL 33016 (US)**
• **GUPTA, Ravinder, K.**
**Pembroke Pines, FL 33024 (US)**

(74) Representative: **Hucker, Charlotte Jane et al**
**Gill Jennings & Every**
**Broadgate House,**
**7 Eldon Street**
**London EC2M 7LH (GB)**

(56) References cited:
WO-A-83/03920          WO-A-92/08134
WO-A-93/15117          US-A- 4 010 038
US-A- 4 452 773          US-A- 4 582 622
US-A- 4 795 698          US-A- 4 891 324
US-A- 5 062 991          US-A- 5 164 311

**Description**

Technical Field

This invention relates generally to colloidal sized particles having an aminodextran outer coating with pendent functional groups attached thereto. Specifically, this invention relates to polymeric colloidal particles having an aminodextran coating that is functionalized to bind a pendant protein such as an antibody, to methods of making such particles and to the use of such particles in biological assays.

Background Art

The use of polymeric particles and magnetic particles to bind a compound has long been known and used in industrial and laboratory procedures. For example, the Merrifield resins, crosslinked styrene-divinylbenzene spheroidal beads, were among the earliest and most widely used modern substrate particles. They were used in organic synthesis, for heterogenizing homogeneous catalysts and in biochemical reactions. Since the Merrifield resins were fairly large, they could easily be separated by filtration. In some fields, however, it is desirable to use colloidal sized particles because the material to be bound is scarce, expensive or is to be used in a procedure wherein larger size particles are not desirable. This is particularly true in the biochemical field.

When particles are of colloidal size, however, their separation from liquid medium by filtration can become lengthy and difficult. In particular, colloidal particles tend to coat the surface of the filter and slow the filtration process. The use of magnetic particles, specifically magnetic particles having a polymeric coating, has found great utility because such particles can be magnetically gathered to one side of a reaction vessel and the bulk of the reaction medium simply decanted. The word "particles" as used herein encompasses spheres, spheroids, beads and other shapes as well and is used interchangeably with such terms unless otherwise specified.

The use of coated magnetic particles has found a particular utility in biological applications, especially where antibodies are bound to the surface coating of the particles. The bound antibodies may be used to capture a specific biological substance from a test sample containing numerous biological samples or to capture undesired species from the test sample, leaving the desired species in the sample.

The categories of coated magnetic particles, also known as magnetic spheres or beads, can be divided into four general classes.

1. Core-and-shell beads with a magnetic core and a hard shell coating of polymerized monomer or a silanizing agent. See U.S. Patent No. 4,267,234 to Rembaum (polyglutaraldehyde shell around ferrofluid core particles); No. 4,454,234 to Czerlinski (suspension or emulsion polymerized coating around submicron magnetic particles); Nos. 4,554,088, 4,695,392 and 4,695,393 to Whitehead et al. (silanized magnetic oxide particles of polydisperse size and shape); No. 4,672,040 to Josephson (polysilane coated magnetic particles); No. 4,783,336 to Margel et al. (suspension polymerized polyacrolein around ferrofluid particles); No. 4,795,698 to Owen et al. (bovine serum albumin coating); and No. 4,964,007 to Yudelson (gelatin-gum arabic-surfactant coating);

2. Core-and-shell beads with a magnetic core and a loose shell of random coil or globular polymer which may or may not be crosslinked. See U.S. Patent No. 4,452,773 to Molday (dextran coating around ferrofluid particles) and No. 4,795,698 to Owen et al. (protein such as bovine serum albumin around ferrofluid particles.

3. Magnetic latex materials formed by uniformly embedding ferrofluid particles in polystyrene latex particles. See U.S. Patent No. 4,358,388 to Daniel et al.

4. Porous polymer particles filled with magnetic materials such as polymer-ferrite or polymer maghemite composite systems. See K. Nustad et al. "Monodisperse Polymer Particles In Immunoassays And Cell Separation", Microspheres: Medical and Biological Applications, A. Rembaum and Z. Tökès, eds. (Boca Raton, Fla.: CRC Press, 1988) pages 53-75; C.D. Platsoucas et al., "The Use Of Magnetic Monosized Polymer Particles For The Removal Of T Cells From Human Bone Marrow Cell Suspensions", ibid. at pages 89-99; and International Patent Publication No. WO 83/03920 to Ughelstad et al. (polymer coated magnetic particles prepared by treating compact or porous particles with a solution of iron salts and the use of such particles for medical, diagnostic or other purposes).

The usefulness of most polymer coated magnetic beads in medical and biological applications has been limited by practical considerations such as the uniformity of particle size and shape, the need for the biological reagent to be strongly bound to the particle, a preference for hydrophilic polymer coatings as opposed to hydrophobic coatings, and whether or not the coating is biodegradable. While biodegradability is of particular importance where a biological reagent is to administered in vivo, it is also important in various cell sorting, separation and assay procedures. The most desirable coated magnetic particles would have the following features.

1. The particles should be as small as possible in order to maximize the surface area on which the biological reagent is coated, but the particles should still be easily separable with a small magnet. Small size and large surface area are desirable in order to use the least possible quantity of particles to remove the targeted substance; e.g., to interact with on the order of $10^6$ cells per sample in one step, thereby avoiding sequential additions and work-ups.

2. There should be a low non-specific binding of the antibody-coated particles to cell surfaces. The particle surface should be hydrophilic or covered with a coating of a hydrophilic substance to which the antibody is attached.

3. The polymer and antibody layers on the particles should be covalently bound to each other in order to reduce dissociation and conformational changes.

4. The coating on the magnetic particles and any molecular chains which link an antibody to the polymer surface should be metabolizable.

5. In positive selection of cells, a mechanism for quickly and easily recovering viable cells from the magnetic particles should be available in order that recovered cells can be cultured.

6. In the negative selection of cells, the antibody-coated particles should be sterile so that the remaining cells can be cultured.

7. For magnetic separation and sorting of cells and other biological substances, the preferred magnetic particles are "soft" magnetic particles. That is, particles which can be easily magnetized and demagnetized as opposed to hard or permanent magnetic. The particles can be ferromagnetic, ferrimagnetic or superparamagnetic. Ferromagnetic and ferrimagnetic particles are not limited in size, whereas superparamagnetic particles are limited to single domain structures of dimensions usually less than about 40 nanometers. (C. Kittel et al., Solid State Physics 3: 437-464 (1956).

Problems exist with using each of the magnetic-composite particles from each of the above class in cell separation procedures. Some examples of the problems encountered are:

1. Ferrofluid core and the usual polymer outer shell particles have too small a magnetic moment to make them practical for use in cell separations where handheld permanent magnets are used to collect and separate the magnetic particles. Such particles require the use high-field separation techniques which severely limits the volume of material which can be processed, thus limiting scale-up.

2. Ferrofluid-polystyrene particles prepared by emulsion polymerization cannot be tightly controlled in size and range from about 0.1 to 4 $\mu$m in diameter. Consequently, in cell separations using antibodies conjugated to such beads, the very small, kinetically-mobile magnetic particles which inherently possess the least magnetic moment tend to preferentially occupy the antigenic sites on a cell surface. As a result, the resulting cell-bead conjugates do not have a sufficient not magnetic moment to permit easy separation.

The use of magnetic particles coated with gelatin is described in WO-A-9208134, and overcomes these difficulties. More specifically, WO-A-9208134 discloses particles having a crosslinked gelatin coating to which are bonded biological substances or molecules, for instance monoclonal antibodies. However, gelatin coated particles have been found to have some problems regarding non-specific interactions with certain cells, notably platelets and phagocyte cells such as monocytes. The problem arises because the amino acid sequence of gelatin (as exemplified by the $\alpha$-1 chain of rat and calf skin collagen) includes three regions with the tripeptide sequence Arg-Gly-Asp (RGD) which duplicates the RGD binding sequence of fibronectin, a component of the extracellular matrix that specifically promotes cellular adhesion. Those biological cells with fibronectin expressed on their surface have a specific affinity for collagen, which is equivalent to crosslinked gelatin. For example, antibody containing gelatin coated magnetic ferrite particles used in the separation of subsets of white blood cells will also bind to fibronectin on the surface of platelets and monocytes. The result is non-specific depletion of cells because monocytes and platelets are bound to the particles as well as those cells which bear antibody-specific antigens. The non-specific depletion of cells can be avoided through the use of an aminodextran as the outermost coating layer on coated particles. The use of dextran derivatives as carriers has been discussed by U. Manabe et al., J. Lab. Clin. Med. 104: 445-454 (1984) (antibody-polyaldehyde dextran-methotrexate); L.B. Shin et al., Intl. J. Cancer 41: 832-839 (1988) (antibody-aminodextran-methotrexate); A.R. Oseroff et al., Proc. Natl. Acad. Sci. USA 83: 8744-8748 (1986) (antibody-aminodextran-chlorine 6); S. Rakestraw et al., Proc, Natl., Acad. Sci. USA 87: 4217-4221 (1990) (antibody-dextran hydrazide-Sn(IV) chlorine 6); R.J. Mrsnay et al., Eur. J. Cell. Biol. 45: 200-208 (1987) (ouabain-aminodextran-gold particle); J.W.M. Bulte et al., Magnetic Res. 25: 148-157 (1992) (anti particle); and other as described in S.S. Wong, "Chemistry of Protein Conjugation and Cross-Linking" (CRC Press, Boca Raton, Florida 1991).

In addition to magnetic particles, there is also a need for polystyrene latex (PSL) particles which have been coated with hydrophilic polymer coatings to which antibodies can be subsequently bound. There polymer coated PSL particles can be used in bead-based cell population analyses and immunoassays. However, non-magnetic PSL particles, as made, usually have a relatively low density of various functional groups such as carboxyl or amino groups. Consequently, covalent coupling of coating materials such as dextran or gelatin to the surface of PSL particles is no satisfactory.

The various particles described above have been used in the biological arts to immobilize a variety of biological substances, particularly antibodies. In using such particles, immobilization of antibodies by covalent coupling is preferred to immobilization by antibody adsorption which requires careful and separate adjustment of pH and antibody concentration for each monoclonal antibody used. P. Bagchi et al., J. Colloid Interface Sci., 83: 460-478 (1981); J. Lyklema, Colloids and Surfaces, 10: 33-42 (1984); M.D. Bale et al., J. Colloid Interface Sci., 125: 516-525 (1988); C. C. Ho et al., ibid., 121: 564-570 (1988); "Proteins at Interfaces: Physicochemical and Biochemical Studies", ACS Symposium Series, No. 343, J.L. Brash and T.A. Horbett, Eds. (Washington: Amer. Chem. Soc., 1987); W. Norde, Adv. Coll. Interface Sci., 25: 267-340 (1986); A.V. Elgersma et al., Abstracts of the 198th Amer. Chem. Soc. Meeting, Miami Beach, Fla., Sept. 10-15, 1989, COLL 0131; and D.E. Brooks, Annenberg Center for Health Sciences and H.B. Wallis Research Facility at Eisenhower Latex Conference, Orlando, Fla., Dec. 4-5, 1989. However, even when the pH and antibody are carefully controlled, there is little assurance that the orientation of adsorbed antibody will be such that an active adsorbed antibody will result. Adsorbed antibodies also have long term storage problems arising from antibody desorption from the particles' surfaces. Furthermore, proteins, such as antibodies, tend to achieve maximum adsorption on hydrophobic surfaces at or near the pI of the protein. However, if electrostatic interactions between charge groups are important, then the adsorbing surface and the adsorbate should have net opposite charges. Covalent coupling methods, on the other hand, are not as sensitive to these conditions.

Covalent coupling methods have been used with particles of magnetite embedded in carboxy-modified latex subsequently coated with aminodextran [R.S. Molday et al. FEBS. Lett., 170: 232-238 (1984)] and derivatized with a number of antibodies. If the antibody is of IgG isotype, the covalent coupling method assures that the linkage between the antibody and the particles occurs at the antibody Fc or hinge region, and not at the antibody's Fab region. If the antibody is of pentameric IgM isotype which has only Fab regions exposed, the coupling of one Fab region to the particle will still leave four Fab regions exposed and available for reaction.

This invention provides for the preparation of magnetic and non-magnetic particles having a biodegradable coating to which can be attached pendent biological substances, such as monoclonal antibodies. The particles of the invention can be used in various cell separation and assay methodologies. Biodegradability in the coating used on the magnetic or latex core material is important in cell separation technology. For example, antibodies may be conjugated to gelatin/aminodextran coated magnetic particles such as manganese ferrite particles. These particles would thus contain a proteinaceous coating and a manganese-iron oxide core, all of which are biodegradable. In a positive cell selection procedure using such particles, once the desired cell has been isolated from other cells, the particles and coating can be allowed to degrade in a manner such that the cells are kept viable and can be cultured for further use. Alternatively, the enzyme collagenase can be used first to release the core material (magnetic or latex) by digestion of the gelatin coating. The core material can then be removed from the cell suspension before culturing the cells. In the negative selection of cells with such biodegradable beads, the beads can be left in the cell suspension from which targeted cells were removed without compromising the viability of the remaining cells. For example, in bone marrow purging operations using biodegradable magnetic beads, there is less concern about leaving behind some beads in the purged marrow that is to be transplanted in a patient. Currently, synthetic polymer-magnetite particles prepared by Ughelstad et al, International Patent No. WO 83/03920, and conjugated with antibody are being used in bone marrow purging. The polymer is not biodegradable and imparts a hydrophobic surface to these beads. This hydrophobicity, which is not present in the gelatin coated particles of the claimed invention, is responsible for non-specific interactions between the beads and cells. As a result of this non-specific interaction, the selectivity is poor and more beads must be used to attain the desired level of treatment. The claimed invention avoids these problems.

Gelatin coated particles have been found to have some problems regarding non-specific interactions with certain cells, notably platelets [Clinical Hematology, 8th ed., M.M. Wintrobe et al., Eds (Lea & Febiger, Philadelphia, Pennslyvania 1981), Chapter 16] and phagocite cells such as monocytes [Basic & Clinical Immunology, 6th ed., D.P. Stites et al., Eds (Appleton & Lange, East Norwalk, Connecticut 1987), Chapter 9]. The problem arises because the amino acid sequence of gelatin (as exemplified by the $\alpha$-1 chain of rat and calf skin collagen) includes three regions with the tripeptide sequence Arg-Gly-Asp (RGD) [The theory of the Photographic Process, 4th ed., T.H. James ed. (Mac Millan, New York 1977) Chapter 2, page 54] which duplicates the RGD binding sequence of fibronectin, a component of the extracellular matrix that specifically promotes cellular adhesion [Biochem. Biophys. Res. Comm 170: 1236 (1990)]. Those biological cells with fibronectin expressed on their surface has a specific affinity for collagen which is equivalent to crosslinked gelatin. For example, the antibody conjugated, gelatin coated magnetic ferrite particles used in the separation of subsets of white blood cells will also bind to the fibronectin that is present on the surface of platelets and moncytes. The result is the non specific depletion of cells from a sample because monocytes and platelets, as well as cells which contain the antibody-specific antigen, will bind to the gelatin coated particles. The non-specific cell depletion can be substantially overcome by using an aminodextran as the outermost particle coating layer.

DISCLOSURE OF THE INVENTION

The invention provides discrete colloidal particles having a core of polymeric substance having a hydrophobic surface coated with a water soluble gelatin or an aminodextran. The polymeric core is non-gelatin, non- aminodextran core selected from the group consisting of polymeric materials or substances which have covalently attached surface amine-reactive groups and polymeric substances which do not have surface amine-reactive groups. A plurality of pendent functional groups is attached to the coated particles. The pendent functional groups may be or have within their structure terminal aldehyde or carboxylate groups, amine groups, sulfhydryl groups or maleimidyl groups, and polyclonal or monoclonal antibodies.

The invention also provides discrete colloidal particles having a solid core, of any substance, and coated with a first layer of a water soluble gelatin and a second layer of an aminodextran, said coating being crosslinked or fixed by the action of a chemical crosslinking agent and having a plurality of pendent functional groups. The pendent functional groups may be or have terminal aldehyde or carboxylate groups, amine groups, sulfhydryl groups or maleimidyl groups, and polyclonal or monoclonal antibodies. The core may be metallic particles formed in gelatin solution or preformed particles which are then coated with the gelatin.

The invention provides discrete colloidal particles having pendent biological functional groups such as polyclonal and monoclonal antibodies covalently attached to the crosslinked gelatin or aminodextran coating by means of a heterobifunctional agent, for example, a short diamine or polyamine chain, so as to enable advantageous use of said antibody functionalized particles in biological separations and assays. The heterobifunctional agent acts as a bridging group between the biological substance or functional group and the crosslinked aminodextran.

The invention further provides a method for the preparation of colloidal particles having a solid core of polymeric material having a hydrophobic surface coated with an aminodextran having a plurality of functional groups. The process, in general, comprises the coating of a solid core polymeric material which has a hydrophobic surface with aminodextran without the formation of covalent bonds between the aminodextran and the polymeric material or coupling the amino groups of an aminodextran to reactive groups on the surface of the polymeric material (for example, aldehyde or carboxylic acid or acid ester groups on the surface of polystryrene particles), crosslinking the adsorbed aminodextran or the coupled aminodextran and derivatizing the crosslinked aminodextran to obtain a product having a desired reactive species covalently bound to said crosslinked aminodextran surface. Though it is preferred to crosslink the aminodextran coupled to the surface of the polymeric beads, such crosslinking is not necessary in all situations. For example, if the biological material under assay or reagents used in the assay do not interact with the polymeric material, crosslinking may not be required. The invention further provides a process for the preparation of particle bound polyclonal and monoclonal antibodies from the aminodextran coated particles, and for the use of such particles in biological assays, particulary immunological assays.

The invention provides a process for the preparation of discrete colloidal particles having a solid core, of any substance, coated with a biodegradable first layer of crosslinked gelatin and biodegradable second layer of an aminodextran having pendent functional groups. The process comprises coating a solid core material which has a hydrophobic surface with first gelatin layer and a second aminodextran layer, crosslinking the adsorbed outer coating and derivatizing the crosslinked coating to obtain a product having a desired reactive species covalently bound to said crosslinked coating surface. The invention further provides a method for the preparation of particle bound polyclonal and monoclonal antibodies.

The invention also provides a process for the separation, either positive or negative, and analysis of biological substances comprising contacting a solution containing a biological substance with an antibody covalently bound to the surface of an aminodextran coated core particle, incubating the resultant mixture at a temperature and for a time sufficient to form a complex between said antibody and said substance, analyzing the resulting particle containing solution to determine particle-shifted light scatter or other particle-shifted properties. If desired, the particles may also be separated from the solution and either or both analyzed independently analysis performed.

BRIEF DESCRIPTION OF THE DRAWINGS

FIG. 1 is a plot of neutrophil depletion as a function of the bead-to-cell ratio used in the depletion process.
FIGS. 2A - 2L are a series of histograms which illustrate the distinct and progressive shift toward less forward scatter (FS) or smaller size and more side scatter (LSS) or greater granularity in the normal graulocyte region as higher titer of magnetic beads were added to the cell samples.
FIG. 3 is a plot of real blood cell depletion as a function of the bead-to-cell ratio used in the depletion process.
FIG. 4 is a plot of platelet depletion as a function of the bead-to-cell ratio used in the depletion process.
FIG. 5 is a plot of white blook cell depletion as a function of the bead-to-cell ratio used in the depletion process.

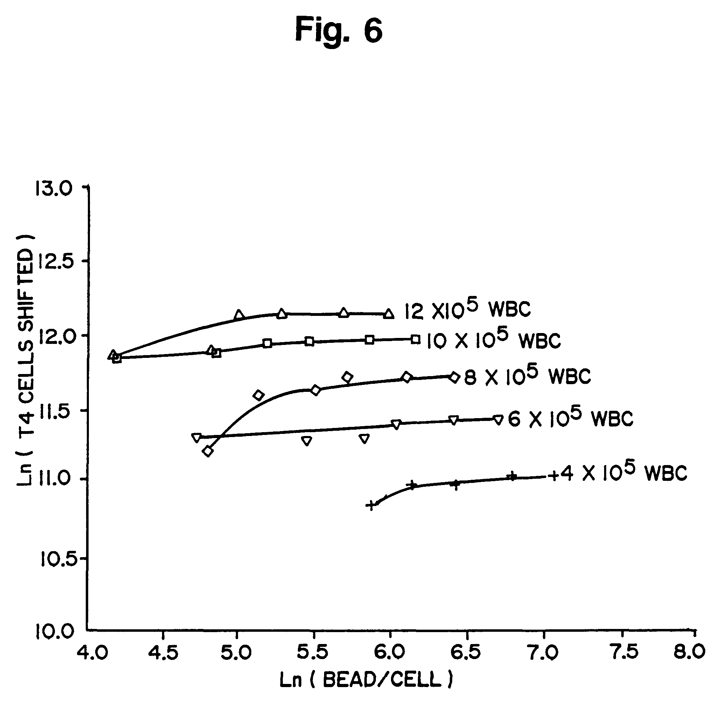

FIG. 6 represents the T4 population shift analysis by T4-antibody, aminodextran coated polystryrene beads as carried out in Example 5.

In the Detailed Description Of The Invention and Preferred Embodiments which follow, applicants place reactive maleimidyl groups on the aminodextran coated particles and reactive sulfhydryl groups on the antibodies. These may be reversed such that the maleimidyl groups are attached to the antibodies and the sulfhydryl groups are attached to the aminodextran. Applicants have also elected to use 2-iminothiolane hydrochloride as the model for the sulfhydryl reagent and sulfo-SMCC (described below) as the model for the maleimidyl reagent. Other reagents enumerated or of like nature and result may also be used.

The aldehyde/sulfate polystyrene latex and sulfated polystyrene latex particles used herein represent two different types of particles. The former has reactive aldehyde groups which can combine with a substance such as an aminodextran and form covalent bonds. The latter lacks such reactive groups. A coating substance is adsorbed onto the surface of sulfated polystyrene particles without the formation of covalent bonds between the adsorbed substances and the particles surface. In addition to polystyrene latex, other, polymeric materials may be used as core particles. For example, polyacrylate, polymethacrylate, polyester, and polyphylene oxide particles, among others, may coated with gelatin or an aminodextran and derivatized as described herein. However, the availability and cost of these other polymeric particles makes the use of polystyrene particles preferred. Other considerations which favor polystyrene are uniformity in the size and shape of the particles which are to be coated. The size of the polymer particles ranges from about 0.1 to about 5.0 microns. The preferred particle size is in the range of about 0.1 to 1.0 microns.

Lastly, the choice of whether or not an aminodextran coating is crosslinked is dependent on two factors. The first is whether or not the aminodextran is conjugated to the polymeric particles surface as it would if, for example, it were coated onto carboxylated beads. If the aminodextran is not conjugated, then the coating must be crosslinked. The second factor is the practitioner's choice. If the aminodextran cotaing is conjugated, then the practioner is free to crosslink the coating or not crosslink it. In general, if a "relatively thick" aminodextran coating is desired, the aminodextran layer conjugated to the polymer surface is crosslinked and additional aminodextran is used to block unreacted crosslinking agent reactive groups. Subsequent reactions are carried out as described herein. The aminodextran/crosslinking agent reactions can be carried out a plurality of time, if so desired. If only a single aminodextran layer is desired, then the layer conjugated to the surface can be further derivatived, without crosslinking, as described herein, or the layer can be crosslinked and unreacted crosslinking agent reactive groups can be blocked by reaction with polyamines such as ethylenediamine and triethylenediamine, amond others. These particles may then be further derivatized as also described herein.

Glossary of Biological Reagents

All references to the monoclonal antibodies (mAb or Ab) used herein are by the identifying designations used by Coulter Corporation, Miami, Florida for monoclonal antibodies made by Coulter Corporation. The following information further identifies the antibodies used herein. The use of these monoclonal antibodies is by way of example only and is not to be understood as limiting the invention. The term "CD" refers to "Cluster Designation" adopted by the International Workshops on Human Leukocyte Differentiation Antigens. A.T.C.C. is the American Type Culture Collection, Rockville, Maryland.

| Antibody | CD | Description or Reference |
|---|---|---|
| T11 | CD2 | Derived from hybridization of mouse NS/1-AG4 cells with spleen cells of BALB/cJ mice immunized with T cell chronic lymphocytic leukemia cells. |
| T4 | CD4 | As T11, but immunized with peripheral human T lymphocytes. |
| T8 | CD8 | As T11, but immunized with human thymocytes. |
| KC16 | -- | U.S. Patent No. 4,752,563; A.T.C.C. Deposit No. CRL 8994. |
| 1D3 | -- | U.S. Patent No. 4,931,395; A.T.C.C. Deposit No. HB 9445 |
| KC48 | -- | U.S. Patent No. 4,865,971; A.T.C.C. Deposit No. HB 9584 |
| MO2 | CD14 | R.F. Todd et al. J. Immunol., 126:1435 (1981). |
| PLT-1 | CD41 | R.F. Todd et al., Blood, 59:775 (1982); Griffith et al., Blood, 61: 85 (1983). |
| KC56 | CD45 | Derived from hybridization of mouse Sp2/0-AG14 myeloma cells with spleen cells from BALB/c mice immunized with a derivative of the CEM cell line. |
| B4 | CD19 | L.M. Nadler et al., J. Immunol. 134:2228 (1985). |

Other reagents used herein and commercially obtainable from Coulter Corporation are:

| | |
|---|---|
| MsIgG1-RD1/MsIgG1-FITC: | Mouse IgG1-phycoerythrin [RD1]/Mouse IgG1-Fluorescein Isothiocyanate [FITC]. |
| T11-RD1/B4-FITC: | Ab T11-phycoerythrin/Ab B4-FITC. |
| T4-RD1/T8-FITC: | Ab T4-phycoerythrin/Ab T8-FITC. |
| 1X PBS: | Dissolve 53.8g $K_2HPO_4$ in 1.6 L distilled water. Add 12.8g $KH_2PO_4$ and stir until dissolved. Then dissolve 340g NaCl in the solution. After all salts dissolved, add distilled water to make up to 2 L volume and filter through a 0.2 $\mu$m filter. The resulting solution in 20X PBS. 1X PBS is prepared by dilution of 1 part 2 PBS with 19 parts distilled water. The 1X PBS solution has a pH in the range of 7.1-7.3, typically 7.2, and is 0.15 M in NaCl. |

Detailed Description

In using the method of the invention, uniform particles (the core material) in the size range of 0.1 to 5.0 microns are coated with aminodextran or gelatin and aminodextran, and the coating is fixed by means of a chemical fixing agent or is chemically bound to the particle surface. The uncoated particles have a hydrophobic or partially hydrophobic surface. The preferred size of the particles is in the range of 0.1 to 1.0 microns.

The magnetic particles used in the claimed invention may be preformed magnetic particles that are dispersible in a gelatin solution or they may be magnetic particles prepared by the in situ use of gelatin in the preparation of said magnetic particles. The in situ method for the preparation of monodispersed colloidal particles of ferrites of manganese, zinc, mixed manganese-zinc, iron, barium, cobalt and nickel involves the use of an aqueous metal hydroxide gel first formed by mixing ferrous and other metal salts in an aqueous gelatin solution with potassium or sodium hydroxide and potassium or sodium nitrate solution, all solutions being purged with nitrogen gas. The conversion of the gel to the metal oxide sol is achieved by mild thermal treatment at 90°C (low temperature) for 4-72 hours, during which nitrate oxidation of ferrous iron occurs. The magnetic particles in the hydrosol are then washed and resuspended in a 1% aqueous solution of gelatin of the type described below prior to further treatment as described herein. In preparing magnetic particles using in situ gelatin as described herein, only one type of gelatin has been found optimal for such use. This is type B or alkali-cured gelatin with a pI range of 4.75 to 5.0. The procedures for the preparation of magnetic particles using in situ gelatin are fully described in US 5062991 and also described herein. The gelatins which are crosslinked according to the present invention are given below.

Gelatin is obtained from highly crosslinked collagen in fibrous tissue, such as skin or bone, which has been acid or base cured and then thermally degraded at or above 39°C. The collagen molecule combines the helical structure of the $\alpha$-type proteins with the inter-chain hydrogen bonding of the $\beta$-type proteins. The three collagen peptide chains, each in the form of a left handed helix, are twisted about each other to form a superhelix. Upon treatment, the three peptide strands of the superhelix are separated by the breaking of inter-chain hydrogen bonds and replacing them with hydrogen bonds to water molecules. The separated peptides have random coil configurations. "The Theory of the Photographic Process", T.H. James,Ed., (New York: MacMillan Press, 1977). The $\alpha$-1 peptide chain has been sequenced and found to have over 1000 residues. D.J.S. Hulmes et al., J. Mol. Biol., 79:137 (1973). They contain extensive segments of mainly non-polar residues; and the polar residues which are present are not localized into acidic or basic regions. Furthermore, in contrast to globular proteins which tend to expose their hydrophilic residues on their surfaces and bury their hydrophobic residues within their structure {see R.E. Dickerson et al., "The Structure and Action of Proteins", (Menlo Park: Benjamin, 1969)}, random coil gelatin has exposed hydrophobic residues readily available for adsorption onto the surface of hydrophobic particles such as polystyrene latex particles or magnetite and ferrite particles. When aqueous gelatin is adsorbed onto the surface of a particle, its hydrophilic side chains (aspartyl, glutamyl and lysyl residues) tend to be directed externally to the aqueous medium. The lysyl groups, which function as the intramolecular crosslinkage points in collagen, will be accessible for cross linking in the adsorbed gelatin. Glutaraldehyde is frequently used as the crosslinking agent. U.S. Patent No. 4,478,946 to Van Der Merwe et al. and S.B. Sato et al., J. Biochem., 100: 1481-1492 (1986).

A number of different, usually bifunctional, crosslinking agents such as bis[2-(succinimidooxycarbonyloxy)-ethyl]sulfone, disuccinimidyl tartarate, ethylene glycol bis (succinimidylsuccinate), disuccinimidyl suberate and glutaraldehyde may be used in the claimed invention. Glutaraldehyde, the preferred gelatin or aminodextran crosslinking agent, as commercially available, contains mainly monomer absorbing at 280 nm (nanometers). However, there is present in the commercial product a significant amount of polymeric material which gives rise to an absorbance at 235 nm. The polymeric species, probably trimers or linear oligomers, are of sufficient length to form infra- and inter-molecular bridges between amino groups present on the adsorbed gelatin. By judiciously selecting the reaction time between the adsorbed gelatin or aminodextran and glutaraldehyde, the gelatin can be suitably fixed on the core particles so that it will not be removed during subsequent separation, reaction and washing steps. Large flocs created by excessive crosslinking of free gelatin can thereby be avoided and interparticle crosslinking is negated.

The types of gelatin suitable for use in the present invention are type A, acid cured, isoelectric point pH 8.3-8.5 and type B, alkali cured, isoelectric point, pH 4.75-5.0. Each type is available in a variety of Bloom Numbers which indicate gel strength. Type A gelatin Bloom Numbers useful in the claimed invention range from 60 to 300. Type B Bloom Numbers useful in the claimed invention range from 60 to 225. The type A, 175 Bloom gelatin used in the preferred embodiment of the claimed invention is preferred and was selected for its relatively large number of lysyl residues and its lower Bloom number in order to minimize intermolecular interactions between gelatin molecules. For optimum adsorption on magnetite and ferrite particles, it was buffered to pH 8.4, the middle of its isoelectric point range, at which pH it is most soluble in water and gives the least viscous solution. The instability of gelatin adsorbed on ferrite particles, which instability arises when glutaraldehyde is added, was overcome by the present invention by the use of more dilute particle and gelatin concentrations [0.1% weight/volume (w/v) instead of the 2.5% w/v solids suspension that was used in other reactions herein in conjunction with an inert polymeric stabilizer, polyvinylpyrrolidone (PVP), that does not react with glutaraldehyde. The use of the stabilizer and the 25-fold lower gelatin concentrations avoids interparticle crosslinking during the glutaraldehyde fixation reaction. Since polymer desorption is a very slow process relative to the time of the glutaraldehyde fixation reaction, approximately 6 minutes, a stable gelatin coating around the core particle was produced.

In order to be useful in the biological and medical arts, the aminodextran or gelatin/aminodextran coating should contain functional groups which can be conjugated with biologically active substances such as antibodies to produce immobilized biologically active substances attached to the particle surface. Covalent coupling of biological substances to the particle surface is preferred over simple adsorption. The coupling of an antibody, either polyclonal or monoclonal, to the crosslinked gelatin surface is accomplished by the use of "short chain" diamines or polyamines and a hetero-bifunctional reagent. (Hereafter, the word polyamine includes diamines). The polyamine is reacted with residual aldehyde or carboxylate groups, either naturally occurring or present by the steps of this invention, present on the crosslinked gelatin surface. The use of polyamine serves not only to block aldehyde/carboxylate groups, but also serves to replenish gelatin amino groups such as lysyl amino groups which were depleted during the crosslinking process. This procedure is generally accomplished in two steps. In the first step, unreacted terminal aldehyde groups are reacted with a polyamine followed by sodium borohydride ($NaBH_4$) reduction of the resulting Schiff's base to create stable, saturated C-N linkages. In the second step, exposed carboxylic acid residues (glutamic, aspartic) of gelatin are coupled to polyamine in the presence of a water soluble carbodiimide such as 1-ethyl-3-(3-dimethylaminopropyl )-carbodiimide (EDAC).

Short chain polyamines, including diamines, are preferred in order to avoid crosslinking neighboring aldehyde or carboxylic acid groups on the same particle or to avoid linking such groups on different particles. One polyamine amine group reacts with the gelatin surface and the other(s) remains unreacted and available for coupling, directly or indirectly, to a biological substance. Examples of 'short chain' polyamines include ethylenediamine, phenylenediamine, propylenediamine, 1,4-cyclohexanediamine, cyclohexenediamine, tetramethylenediamine, diethylenetriamine, 1,5-diamino-3-(2-aminoethyl)pentane $[(H_2NCH_2CH_2)_3C]$ and other polyamines of general formulas $H_2NCH_2$-$(CH_2)_x$-$CH_y$ $(CH_3)_z$-$NH_2$ and $C_6H_{4+a}$ $(NH_2)_{21}$ where x= 0-3, y= 1 or 2 and z= 1 when y= 1 or z=0 when y=2, and a= 0 or 6. Ethylenediamine is preferred.

The coupling of the biological substance to the particle involves activation of the free amino groups of the coated, crosslinked particles with a water soluble hetero-bifunctional reagent such as 2-iminothiolane hydrochloride (IT), sulfosuccinimidyl-4-(N-inaleimidomethyl)cyclohexane-1-carboxylate (sulfo-SMCC), m-maleimidobenzoyl-N-hydroxysuccinimide ester, N-succinimidyl-3-(2-pyridyldithio)propionate, succinimidyl-4-(p-maleimidophenyl )butyrate, N-succinimidyl-(4-iodoacetyl)aminobenzoate, the reagents listed above as substitutes for glutaraldehyde and the like. The 2-iminothiolane hydrochloride and the maleimidyl/succinimidyl reagents are preferred. E.Ishikawa, Immunoassay Supp., 1:1-16 (1980) and J. Immunoassay, 4:209-227 (1983); M. Imagawa et al., J. Appl. Biochem., 4: 41-57 (1982); and M.D. Partis, J. Protein Chem., 2: 263-277 (1983). When using sulfo-SMCC, the active sulfosuccinimidyl ester end of sulfo-SMCC will react at pH 7.0-7.5 with amines to give peptide bonds. The sulfo-SMCC/diamine bridging unit which results is approximately 16 Angstroms in length.

When performing the polyamine and sulfo-SMCC reactions, particle aggregation was monitored by microscopic examination (1000x magnification) and by light scattering analysis using a Coulter N4MD submicron particle sizeanalyzer (Coulter Corporation, Miami, Florida), or similar instrument.

The maleimidyl group of sulfo-SMCC will react at pH 6.5-7.5 with free sulfhydryl groups to form a stable, covalent thioether bond. However, it is essential that the coated particles with which sulfo-SMCC is reacted contain no free sulfhydryl groups which could react with the maleimidyl end of sulfo-SMCC. Sulfhydryl groups are found on or generated from cystine and cysteine amino acid residues of which gelatin has very few. Consequently, when gelatin is used as first coating there is no need for a protein modifier to block free sulfhydryl groups prior to reaction with sulfo-SMCC.

Biological substances, particularly either monoclonal or polyclonal antibodies, can be covalently linked to the maleimidyl end of sulfo-SMCC functionalized particles by means of sulfhydryl groups present, either naturally or by derivatization, on said biological substances. Biological substances which have cysteinyl residues inherently contain sulfhydryl groups. To introduce additional sulfhydryl groups, the biological substances' amine groups are activated with Traut's reagent, 2-iminothiolane hydrochloride (IT), at a pH in the range of 7-10. M. Erecinska, Biochem. Biophys. Res. Commun., 76:495-500 (1977); J.M. Lambert et al., Biochemistry, 17: 5406-5416 (1978); and M.E. Birnbaumer et al., Biochem J., 181: 201-213 (1979). When the bio-substances are antibodies, antibody lysyl and terminal amine groups are activated by IT. In the present invention, reaction conditions and the concentration of reactants were varied to determine the optimal coupling so that the bio-substance, especially antibody, when conjugated with the substrate particles, retains its maximum functional activity. Although maleimides react quite rapidly with sulfhydryl groups in solution, the same groups immobilized on particles were given longer reaction periods to react with protein. Particle and antibody concentrations during antibody conjugation were optimized to avoid aggregation, particularly when IgM antibodies were used. The procedures optimized for IgM antibodies can be used for all monoclonal antibodies with an isoelectric point range of about 5.0 to about 9.0. Generally, about 30-fold less antibody was required to achieve covalent coupling than is required for simple adsorption; a consequence of importance where expensive or hard to obtain antibodies are involved.

The optimum concentration of iminothiolane-activated antibody to use in conjugation reactions with maleimidyl-activated particles was determined by the use of activated antibody binding curves (Surface Antibody vs Total Antibody Concentration). After a typical conjugation period, a sample is taken and filtered through a 0.2 $\mu$m low-protein binding filter. The filtrate is analyzed spectrophotometrically and the surface antibody is determined by the difference between the total antibody in the starting solution and the antibody in the filtrate (Total Antibody - Filtrate Antibody). The binding data in antibody (Ab) concentration dependent runs show Langmuir isotherm-type characteristics; i.e., a linear low concentration region for total antibody versus surface antibody concentration, a smooth inflection point and a plateau indicating saturation at the particle surface at high concentrations. The antibody concentrations actually used were those at the inflection point or at concentrations slightly above the inflection point. Binding constants were obtained graphically by recasting the equation of a hyperbola into one for a straight line. A double reciprocal plot of $1/n_2^s$ versus $1/C_2$ was constructed, where $n_2^s$ is the number of moles of IT-Ab bound per gram of particles and $C_2$ is the molar concentration of free IT-Ab at equilibrium. Linear plots are indicative of Langmuir-type binding behavior. The binding constants $K_1 = n^sK$ of IT-Ab for sulfo-SMCCactivated ferrite particles were calculated using the equation $1/n_2^s = 1/(n^sKC_2) +1/n^s$, where K is the intrinsic binding constant and $n^s$ is the number of moles of binding sites per gram of ferrite particles. Linear regression analysis of plots for various monoclonal antibodies gave the following results:

| Ab T11 | $K=1.3 \times 10^6 M^{-1}$ | $n^s = 5.9 \times 10^{-8}$ mol/g |
|---|---|---|
| Ab KC16 | $K = 6.4 \times 10^6$ " | $n^s = 5.1 \times 10^{-7}$ " " |
| Ab 1D3 | $K = 2.7 \times 10^6$ " | $n^s = 2.0 \times 10^{-7}$ " " |
| Ab MO2 | $K = 1.8 \times 10^7$ " | $n^s = 7.1 \times 10^{-7}$ " " |

The results for the ferrite particles compare favorably with similar data for commercially available carboxy-modified latex beads (23% magnetite, 0.980 $\mu$m dia., obtained from Rhône-Poulenc) covalently coated with aminodextran and conjugated to monoclonal antibodies and protein. These results are:

| Ab T11 | $K = 6.5 \times 10^5 M^{-1}$ | $n^s = 1.1 \times 10^{-7}$ mol/g |
|---|---|---|
| Ab KC16 | $K = 3.2 \times 10^6$ " | $n^s = 6.9 \times 10^{-8}$ " " |
| Ab 1D3 | $K = 3.2 \times 10^5$ " | $n^s = 1.7 \times 10^{-7}$ " " |
| Ab MO2 | $K = 2.0 \times 10^6$ " | $n^s = 1.6 \times 10^{-7}$ " " |
| Ab KC48 | $K = 2.5 \times 10^5$ " | $n^s = 7.6 \times 10^{-8}$ " " |
| Ab PLT-1 | $K = 2.8 \times 10^5$ " | $n^s = 2.2 \times 10^{-7}$ " " |
| Streptavidin | | |
| | $K = 1.3 \times 10^6$ " | $n^s = 9.5 \times 10^{-8}$ " " |

In addition to ferrite core beads, the present invention was also evaluated using monoclonal antibodies conjugated to crosslinked gelatin-coated polystyrene beads. The binding constants for these antibodies, which compare favorably to both evaluations given above, are:

| Ab T8; | $K = 1.7 \times 10^6 M^{-1}$ | $n^s = 9.5 \times 10^{-8}$ mol/g |
|---|---|---|
| Ab T4; | $K = 2.5 \times 10^7$ " | $n^s = 3.5 \times 10^{-8}$ " " |

The results with the polystyrene beads indicate that the method of the present invention is not limited to magnetic spheres, but may be used with any colloidal particles that have a hydrophobic surface.

Examples

I. PREPARATION OF MAGNETIC PARTICLES HAVING GELATIN COATING (for Reference only; not an Example of the Invention)

Preparation of Magnetite and Other Magnetic Particles in Gelatin Solution.

10 mmol (5 ml) of 2M $KNO_3$ solution, 12.5 mmol (2.5 ml) of 5M KOH solution and 11.25 ml of double distilled water (DDW) were mixed and purged with $N_2$ gas for 10 minutes (Solution A). 6.25 mmol (6.25 ml) of 1M $FeSO_4$ solution and 25 ml of freshly prepared, $N_2$ purged, 2% type B, 225 Bloom, bovine skin gelatin solution [useful gelatin solution range is from about 0.8% to about 2.0%] were then added to Solution A in a Pyrex® bottle, mixed, swept with $N_2$ gas, capped tightly, and placed undisturbed in an oven at 90°C for 4 hours. After the suspension of black magnetite particles had reached room temperature, they were sonicated for 1/2 hour, washed with 1% type B, 225 Bloom gelatin solution, and then contacted with a large excess of 1% w/v gelatin as is the next step.

Metal ferrites may also be prepared using gelatin in situ in their preparation. In trials with other metals, namely $Mn^{2+}$, $Zn^{2+}$, $Co^{2+}$, $Ni^{2+}$, and ($M^{2+}$), the molar ratio of $M^{2+}$ :$Fe^{2+}$ was kept at 1:2, but nitrate was used instead of sulfate for $Co^{2+}$ and $Ni^{2+}$. The total metal-to-hydroxide molar ratio was maintained at 1:2; but the relative $KNO_3$ to total metal and $KNO_3$ to KOH molar ratios were altered. In preparing the mixed Mn/Zn ferrite, a 1:1 molar ratio of manganese sulfate to zinc sulfate and the same total molar amount of non-ferrous metal ions were used. The following is an example.

10 mmol (5 ml) of 2M $KNO_3$ solution, 18.75 mmol (3.75 ml) of 5M KOH solution and 6.875 ml DDW were mixed and purged with $N_2$ gas for 10 minutes (Solution C). 6.25 mmol (6.25 ml) 1M $FeSO_4$ solution, 3.125 mmol (3.125 ml) of 1M $Co(NO_3)$ solution and 25 ml of type B, 225 Bloom, bovine skin gelatin solution were mixed and purged with $N_2$ gas for 10 minutes. (Solution D). Solution D was added to Solution C in a Pyrex® bottle, mixed, swept with $N_2$ gas, capped tightly, and placed undisturbed in an oven at 90°C for 5 hours. After the suspension of brown particles had reached room temperature, they were sonicated for 1/2 hour, washed with 1% type B, 225 Bloom gelatin solution and then contacted with a large excess of 1% w/v gelatin as in the next step.

Using the methods described above, cobalt and nickel ferrite particles of about 0.1 and 0.2 μm in diameter and of spherical shape were formed in large, loosely-held brown aggregates. Zinc gave low yields of light brown magnetic material of less than 0.2 μm diameter even after 72 hours of heat treatment. Dark brown manganese ferrite particles of uniform, spherical shape and 0.3 μm diameter were obtained as single particles in 83-88% yields. Similar light brown manganese-zinc ferrite particles were produced in 49-55% yield after 72 hours of heat treatment at 90°C. For barium, the procedure was modified since $BaSO_4$ is insoluble in water. (Except for the case where barium is present, the divalent metals may be used as their chlorides or sulfates as well as their nitrates). Thus 6.25 mmol (6.25 ml) of 1M $FeCl_2$ solution, 0.5 mmol (5.0 ml) of 0.1 $Ba(NO_3)_2$ solution and 25 ml of 2% gelatin were mixed and purged with $N_2$ gas for 10 minutes (Solution D). Solution C and the remainder of the ferrite preparation procedure was unchanged except 10 mmol KOH solution (2 ml) was used and the heat treatment was continued for 20 hours. Black barium ferrite particles of uniform non-spherical shape with a 0.2 μm diameter were produced.

Preparation of Gelatin Coated Magnetic Particles.

A quantity of magnetic particles, for example, manganese ferrite particles, of uniform size (0.3 μm) and spherical shape and prepared using in situ gelatin according to the procedures described above were contacted with a large excess of 1% w/v, type B, 225 Bloom aqueous gelatin solution. Alternately, preformed (i.e., formed by methods other than the in situ use of gelatin), dispersible magnetic particles, for example, manganese ferrite particles, of uniform size (0.3 μm) and spherical shape were contacted with a large excess of 1% w/v, type B, 225 Bloom gelatin solution at ambient temperature for approximately 60 minutes. The particles (either of the above) were then magnetically separated and washed five times with a 2% w/v, type A, 175 Bloom gelatin solution in 0.2M aqueous sodium chloride, pH 8.4. After washing, the particles were stored at ambient temperatures for up to several months as 2.5% w/v (weight/volume) solids suspension in a 2% w/v aqueous solution of the type A gelatin containing 0.2M sodium chloride, 0.1% w/v sodium azide at pH 8.4. Provided the azide content of the storage solution is maintained, the suspension can be stored for up to about 3 months.

Crosslinking the Adsorbed Gelatin.

62.5 μL of 25% aqueous glutaraldehyde (0.156 mmol) solution were added to 120 ml of 1% aqueous polyvinylpyrrolidone (MW= 40,000) in 0.2M aqueous sodium chloride, pH 7.2. To this, 5 ml of the 2.5% solid suspension prepared above were added to the glutaraldehyde solution and the resulting suspension was mixed at ambient temperature for a time in the range of 3-15 minutes, preferably about 6 minutes.

Blocking of Unreacted Aldehyde Groups.

0.105 ml of 99% ethylenediamine (1.56 mmol) was added to a 125 ml suspension of the fixed, gelatin coated magnetic particles (0.1%w/v solids) in 1% PVP solution, 0.2M in sodium chloride, pH 7.2. The resulting suspension was mixed for a time in the range of about 1 to 4 hours, preferably about 2 hours, in a 250 ml tissue culture flask. At the end of the mixing time, 1.25 ml of a 10 mg/ml solution of sodium borohydride ($NaBH_4$) in 0.1 mM KOH were added to the magnetic particles and the resulting suspension was mixed for an additional 15 minutes. The particles were then magnetically separated and washed a plurality, preferably three, times with 0.2M aqueous sodium chloride.

Crosslinking the Adsorbed Gelatin Without the use of Polyvinylpyrroilidone as a Stabilizer.

5 ml of 2.5% w/v solids manganese ferrite particles suspended in 2% w/v type A, 175 Bloom gelatin in 0.1M phosphate buffer, pH 8.4, which were prepared as described above were magnetically separated. The clear supernatant liguid was discarded and the residue of magnetic particles was resuspended in 5 ml of 3 mg/ml glutaradehyde solution prepared by mixing 56 µL of 25% aqueous glutaraldehyde solution with 5 ml of 1 mN aqueous potassium hydroxide, pH 10.00. The resulting suspension of magnetic particles was mixed, preferably roller mixed, for about 30 minutes. After the glutaraldehyde addition and mixing was completed, about 34 µL of ethlylenediamine (10:1 diamine-to-glutaraldehyde molar ratio) were added to the reaction mixture which was then stirred for an addiitonal 2-3 hours. Subsequently, about 0.313 ml of a 40 mg/ml solution of sodium borohydride in 1 mM KOH was added to the reaction and the resulting mixture stirred for about 10-30 minutes. The crosslinked particles were then washed three times using magnetic separation and resuspended in 5 ml of 1 mM aqueous potassium hydroxide.

For elemental analysis, a 5 ml suspension of the crosslinked gelatin on manganese ferrite particles was further washed fifiteen times (15) with distilled water and the magnetically separated residue was dried at 100°C in an oven. Analysis in weight percent was: Mn, 19.05%; Fe, 49.49%; C, 0.54%, H < 0.5%; 0, 30.92% by difference. The percentage carbon by weight in gelatin can be obtained from its amino acid content which gives: C, 50.46%; H, 6.765%; N, 18.27; 0, 24.29% and S, 0.21%. [See "The Theory of the Photographic Process", 4th Ed., T.H. James, ed. (Macmillan, New York 1977), Chapter 2, page 52]. Thus, for 1 gram of ferrite-gelating particles, there will be 1 g x 0.0054/0.5056 = 0.01070 gram gelatin and 0.9893 gram ferrite. The volume per particle of sherical 0.29 µm diameter manganese ferrite particles is $1.277 \times 10^{-14}$ cm$^3$. Using a particle density of 4.24 g/cm$^3$, the mass per particle is $5.414 \times 10^{-14}$ gram and the number of particles in 0.9893 gram ferrite is $1.827 \times 10^{13}$. Thus, the mass of gelatin per particle is $5.856 \times 10^{-16}$ gram. Assuming a density of gelatin coating of 0.02 g/cm$^3$ from the 2% w/v gelatin before crosslinking with glutaraldehyde, the volume of gelatin per particle is $2.92 \times 10^{-5}$ cm$^3$. Thus the total volume (ferrite and gelatin) per particle is $4.205 \times 10^{-14}$ cm$^3$ and the radius of a phere consisting of ferrite and gelatin is $2.157 \times 10^{-5}$ cm or 0.2157 µm. The thickness of the gelatin coating is then 0.2157 µm - 0.145 µm (mean radius of ferrite particle) = 0.0707 µm (i.e., about 71 nm). This value for the thickness of gelatin coating is in good agreement with the following adsorption data for gelatin on various surfaces as obtained from the following publications.

1. A.T. Kudish et al., Proteins at Intergaces, ACS Symposium Series 343, T.L. Brash and T.A. Horbett eds. (Washington, D.C.; American Chemical Society 1987), Chapter 17, pages 261-277: Type A, pig skin gelating on glass gave a coating thickness of 750 Å (0.075 µm) and Type B, calf skin gelatin on glass gave a coating thickness of 500 A (0.050 µm).
2. N. Kawaniski et al., J. Phys. Chem. 94: 4611-4617 (1970): Type B gelatin, Bloom 259 on mica gave a force versus distance (between mica surfaces) attractive minimum at 75 nm (750 Å).
3. H. Meltger et al., J. Coll9oid Intergace Sci. 126: 292-303 (1988): Denatured, soluable calf skin collagen (i.e., gelatin) on glass gave film thickness of 600-700 A (0.06000 - 0.0700 µm).

When particles are to be further used and not used just for analysis, the residual aldehyde groups from the glutaraldehyde are removed by reaction with a diamine. Reaction with Fixed Gelatin's Carboxylate Residues.

2.11 ml of 99% ethylenediamine were added to an 118 ml suspension of the aldehyde-blocked beads, 0.1% w/v solids, in 0.2M aqueous NaCl. The resulting suspension was physically and sonically mixed for approximately 15 minutes. After this mixing, 4.5 ml of 10 mg/ml EDAC in 0.2M NaCl was added and the suspension was first physically and sonically mixed for approximately 15 minutes, and finally physically mixed for a time in the range of about 8-16 hours. The contents of the flask were then magnetically separated, washed a plurality of times with 1X PBS, sonically mixed in 1X PBS for approximately 30 minutes, and finally concentrated to 5 ml of 2.5% w/v solids in 1X PBS. For large scale (100x) preparations, the previous aldehyde blocking step and the EDAC coupling step have been combined to avoid multiple separations and washings. The combination of steps did not result in any loss of activity in the final antibody-conjugated beads.

Activation of Diamine Treated Particles with Sulfo-SMCC.

In general, 27 μL of freshly prepared 10 mg/ml sulfo-SMCC in 1X PBS were used per milliliter of 2.5% w/v magnetic particle suspension. In a typical preparation, 135 μL of the sulfo-SMCC solution was added to 5 ml of 2.5% w/v particles. The mixture was then roller mixed in a 15 ml plastic centrifuge tube for approximately one hour, sonically mixed for approximately 5 minutes, magnetically separated, and washed a plurality of times with 1X PBS.

The functionalized, crosslinked, gelatin coated particles resulting from the above series of steps have pendent maleimidyl groups and are suitable for a variety of medical and/or biological uses. If the substance which is desired to be conjugated to the particles has a sufficiency of active sulfhydryl groups, activation of that substance is not necessary, and the following step may be skipped.

Antibody Activation with 2-iminothiolane Hydrochloride.

A 51.24 mg/ml concentrate of T11 monoclonal antibody in 1X PBS containing 0.1% $NaN_3$ was prepared. For 10 mg of T11 antibody and 15 mg/ml antibody concentration during coupling, the total reaction volume should be 0.667 ml. Using a 15:1::IT:T11 activation ratio, 0.9375 μmol (0.129 mg) IT (65 μL of 2mg/ml IT) in 1X PBS is required. Therefore, 0.407 ml of 1X PBS solution was added to 0.195 ml of T11 concentrate, to which resulting solution an additional 65 μL of 2mg/ml IT solution was added. The net resulting solution was roller mixed in a tube reactor for 1 hour. The content of the reaction tube was then applied to the top of a 20 ml G-50 Sephadex column, equilibrated and washed with 100 ml 1X PBS. The derivatized antibody was eluted using 1X PBS and a plurality of 2.5 ml fractions were collected with the aid of a UV monitor. Fractions in the middle of the band absorbing at 280 nm were pooled and the $A_{280}$ value was used to determine T11/IT antibody concentration. Typically, the T11/IT concentration was about 3.0 mg/ml. The T11/IT solution may be concentrated by solvent removal.

Conjugation of T11/IT with Sulfo-SMCC Derivatized Particles.

In a laboratory scale conjugation, total volume 5 ml, the concentration of particles was 2.5% w/v solids and the T11/IT concentration was 0.9 mg/ml. In one sample, when the purified T11/IT solution concentration was 1.850 mg/ml, then 2.392 ml of T11/IT antibody solution in 1X PBS was added to 5 ml of 2.5% w/v solids sulfo-SMCC activated particles which had been preconcentrated by the removal of 2.432 ml of supernatant. The T11/IT solution was added to the particles in 0.5 ml increments with sonic and rapid physical mixing between additions. The resultant solution was then roller mixed in a 15 ml tube for approximately two hours. A 1 ml test sample was then taken, filtered through a low-protein binding 0.2 μm filter, and the filtrate analyzed spectrophotometrically for T11 antibody by measuring the absorbance at 280 nm; $A_{280}$ = c (supernatant) = 0.3986 mg/ml. [Measurement by difference, c (surface) = c (total) - c (supernatant)]. Thus c (surface) = 0.9 mg/ml - 0.3986 mg/ml = 0.501 mg/ml. This translates to a T11 surface loading of 20 mg T11 per gram particles or, for a specific surface area of 4.89 $m^2$/g for manganese ferrite particles, a 4.1 mg T11/$m^2$ particle surface area. Similar procedures with 2- and 3-fold dilutions of particle concentration, but the same total antibody concentration during conjugation, gave higher surface antibody loading. However, a limitation was reached when a 4-fold dilution of the particles concentration did not yield higher surface coverage of antibody.

Blocking Unreacted Maleimidyl and Sulfhydryl Groups.

Unreacted maleimidyl groups on the sulfo-SMCC activated particles were blocked with L-cysteine after antibody conjugation. Typically, 0.480 ml of 5 mg/ml L-cysteine in 1X PBS was added to remaining 4 ml of the conjugation mixture of the previous step and the resulting solution was roller mixed for 15 minutes. Unreacted sulfhydryl groups were blocked by the addition of 0.534 ml of 20 mg/ml iodoacetamide in 1X PBS followed by the addition of 0.100 ml of 1M, pH 9.8 sodium borate buffer solution. The resulting solution was roller mixed for 30 minutes, the blocked conjugation mixture was magnetically separated and the particles washed three times with 1X PBS containing 1% bovine serum albumin (fraction V, heat shock) and 0.1% $NaN_3$ (BSA buffer solution). After washing, 4 ml of the foregoing BSA solution were added to the particles, the particles roller mixed for approximately 1 hour, stored at 4°C for a time in the range of about 8-16 hours, magnetically separated and washed three additional times with BSA buffer.

Antibody containing particles prepared according to the method described herein have been found useful in various cell separation assays. The biological substances used in assays utilizing the invention may be selected from the groups consisting of normal or non-normal T-cells, B-cells, leukocytes, viruses, erythrocytes, cells of the breast, uterus, colon, kidney, liver, lung, testes, stomach, thyroid and parathyroid, and the like; provided that the biological substance contains an antigenic determinant capable of binding to an antibody.

In an embodiment of the invention equivalent to the magnetic particle embodiment described above, the maleimidyl groups and the sulfhydryl groups are transposed. That is, the crosslinked gelatin coated particles are derivatized to have pendent groups ending in reactive sulfhydryl groups in place of the maleimidyl groups described above and the antibodies are derivatized to have reactive maleimidyl groups in place of the sulfhydryl groups described above. The methods used to prepare this equivalent embodiment are the same as described above. In both cases, the antibody is connected to the gelatin surface by a molecular bridge prepared as described.

The following examples are given to illustrate the utility of the claimed invention and are not to be taken as limiting said invention.

Reference Example 1. Protocol for Magnetic Bead Depletion of T-cell and B- cell Populations.

Mononuclear cells (MNC) were obtained from whole blood samples by density isolation on Ficoll-hypaque gradients and washed in PBS. $1 \times 10^6$ MNC in 1 ml 1X PBS were added to a series of tubes containing 5, 10, 25, 50 and 100 $\mu$L of the monoclonal antibody (mAb) conjugated magnetic particle suspension (2.5% w/v) being tested. Two tubes were set up for each depletion and for the undepleted control. The resulting suspensions were then nutated for 3 minutes in a multi-tube vortexer or a single tube nutator. At the end of incubation, the cell suspension was placed for a total of 2 minutes in the magnetic field provided by a single tube magnetic rack. At the end of the magnetic separation, unbound cells were extracted by withdrawing all the clear liquid from the center of the tube with a Pasteur pipet.

For T- or B-cells (T11, T3, T4, TB, B1, B4), the cell suspension collected after depletion was compared directly to the original cell suspension prior to particle depletion. The samples, original and depleted, were centrifuged for 5 minutes at 1200 rpm and the supernatant decanted to leave approximately 100 $\mu$L of 1x PBS remaining in each tube. One tube of each pair of depletion tubes was then stained with 10 $\mu$L CYTO-STAT® MsIgG1-RD1/MsIgG1-FITC control reagent (MS) and the other tube was stained with 10 $\mu$L CYTO-STAT® T11-RD/B4-FITC reagent (for T11, T3, B1 or B4 depletions) or with 10 $\mu$L of T4-RD1/T8-FITC reagent (for T4 or T8 depletions) at room temperature for 10 minutes. At the end of incubation, 500 $\mu$L of PBS were added to each sample and the samples were analyzed by flow cytometry. The samples ware analyzed on the EPICS® Profile using the MBead 2-Color program. (EPICS® and CYTO-STAT® are registered trademarks of Coulter Corporation). As the original sample stained with Ms control reagent was being run, it was checked to determine whether the lymphocyte population was fully incorporated in Bitmap 1, and adjustments were made if necessary. The left side of discriminator 2 was set for each fluorescence histogram on the channel which would give <1% positive staining. This was done for each sample stained with Ms control reagent and then the corresponding tube stained with specific antibody was analyzed. The data were collected and recorded as the absolute number of positive staining cells in the red and green histograms (T and B or T4 and T8) not percent positive. Test results are summarized below.

Reference Example 2. Protocol for Magnetic Bead Depletion of Red Blood Cells (RBC).

100 $\mu$L of $Na_4$ EDTA-anticoagulated whole blood was placed in a series of reaction tubes. To each tube, 25 to 150 $\mu$L of KC-16 conjugated magnetic particles suspension (2.5% w/v) were added and the total volume was adjusted to 250 $\mu$L using PBS. The suspension were nutated for 3-5 minutes in a multitube vortexer or a single tube nutator at low mixing speed. When nutation was completed, 1 ml of PBS was added to each sample tube which was then placed on a magnetic rack for 2-5 minutes. All the supernatant was removed from each tube using a Pasteur pipet and saved in labelled tubes. Samples were analyzed on a Coulter S-plus® IV or similar rbc counter as total rbc number/ml whole blood. The positive control was 100$\mu$L whole blood plus 1.150 ml 1X PBS to give 100% rbc count and the negative control was 100 $\mu$L whole blood plus 1.150 ml of Batch lyse or similar lysing agent to give 0% rbc count. Percentage of rbc depleted= 100% [(rbc count in sample tube)/(100% rbc count)].

Reference Example 3. Protocol for Magnetic Bead Depletion of Leukocytes

100 ml of $Na_4$ EDTA-anticoagulated whole blood were collected, divided among a number of centrifuge tubes and centrifuged at 500g for 10 minutes. The majority of plasma was removed and the buff colored layer of cells from each tube was removed, pooled together and centrifuged at 500g for an additional 10 minutes. The buff colored cells and the plasma constitute the leuko-rich whole blood which should have an rbc count no greater than $8.0 \times 10^9$/ml and a white blood cell (wbc) of $2-4 \times 10^7$/ml.

100 $\mu$L of leuko-rich whole blood were pipetted into a number of reaction tubes. An amount of 10 to 160 $\mu$L of magnetic bead suspension (2.5% w/v) was then pipetted into each tube followed by the addition of 200 $\mu$L of 1X PBS. (N.B. Lower titer points with 10 to 40 $\mu$L of beads should be run first. Additional beads were added only if endpoint depletion was not obtained at 40 $\mu$L). Each tube was nutated for 3-5 minutes at low speed. 2 ml of 1X PBS were then added, the contents of a tube mixed and then magnetically separated for 2 minutes. All supernatant liquid was removed and placed in a duplicate tube which was then centrifuged at 400g for 5 minutes. The resulting supernatant was then carefully removed by pipette and analyzed.

The leuko-rich or the leuko-depleted whole blood samples were analyzed by the addition of 10 μL of single or dual color antibody preparation designed to discriminate for the depletion of specific cells from a mixture of cells. For example, when T11-conjugated magnetic beads were used in depletion, T11-B4 dual color was used to discriminate between actual T11 cell depletion and the non-speciic depletion of T11 cells (i.e. B cells). The mixture was vortexed and incubated for 10 minutes at room temperature in the dark. Controls were isotype control and antibody control with undepleted cells. The tubes were then placed on a Coulter EPICS® Q-prep, or similar instrument, and run on the 35 seconds lyse mode. After the rbcs were lysed and the samples fixed (Q-prep), all samples were analysed on a Coulter EPICS® Profile flow cytometer or similar instrument. This procedure is required to obtain data as actual number of cells per volume of sample. Programs available on Profile were used to analyze lymphocyte and monocyte-myeloid populations. μL: $4.5 \times 10^6$ wbc, $1 \times 10^7$ rbc and $4.3 \times 10^7$ platelets; and 80 μL: $4.5 \times 10^6$ wbc, $1 \times 10^7$ rbc and $4.3 \times 10^7$ platelets. The results indicate that 40 μL of 2.5% solids beads which contained $1.85 \times 10^{10}$ particles removed $4.3 \times 10^8$ rbc, thus giving a particle-to-rbc ratio of 43.

4. In a myeloid cell assay, the undepleted control gave 73,821 lymphocyte, 13,426 monocyte and 55,661 granulocyte counts. Depletion studies were conducted using 10, 20, 30 and 40 μL of 2.5% w/v solids magnetic beads conjugated with KC-48 antibody. The results were 10 μL: 70,330, 9,309 and 340 counts; 20 μL: 68,414, 2,006 and 1,332 counts 30 μL: 62,966, 1,597, and 922 counts; and 40 μL: 59,340, 1,546 and 899 counts, all respectively.

A similar depletion study was conducted using 10, 20, 30 and 40 μL of of 2.5% w/v solids magnetic beads conjugated with 1D3 antibody. The results were 10 μL: 76,405, 13,839 and 1,597 counts; 20 μL: 73,198, 8,653 and 1,216 counts; 30 μL: 65,667, 2,590 and 2,130; and 40 μL: 66,276, 1,906 and 1,686 counts, all respectively.

A further depletion study was conducted using 10, 20, 30 and 40 μL of 2.5% w/v solids magnetic beads conjugated with MO2 antibody. The results were 10 μL: 72,563, 3,107 and 56,520 counts; 20 μL: 72,905, 3,616 and 34,533 counts; 30 μL: 69,644 1,618 and 32,313 counts; and 40 μL: 69,477, 1,210 and 30,899 counts, all respectively.

5. In an erythrocyte/thrombocyte assay, the undepleted control contained $7 \times 10^6$ wbc, $4.9 \times 10^{10}$ rbc and $3.0 \times 10^7$ platelets. Depletion studies were conducted using 20, 40, 60 and 80 μL of 2.5% w/v solids magnetic beads conjugated with PLT-1 antibody. The results, after depletion, were 20 μL: $10 \times 10^6$ wbc, $5.4 \times 10^{10}$ rbc and $1 \times 10^6$ platelets; 40 μL: $10 \times 10^6$ wbc, $5.8 \times 10^{10}$ rbc and $1 \times 10^6$ platelets; 60 μL: $7 \times 10^6$ wbc, $5.1 \times 10^{10}$ rbc and $1 \times 10^6$ platelets; and 80 μL: $10 \times 10^6$ wbc, $5.6 \times 10^{10}$ rbc and 0 platelets.

II. PREPARATION OF MAGNETIC PARTICLES HAVING A FIRST GELATIN LAYER AND A SECOND AMINODEXTRAN LAYER

Preparation of Aminodextrans.

Method A. Small scale preparation of aminodextran.

Aminodextran was prepared by partial cleavage and oxidation of the glycopyranose rings in dextran to aldehyde functional group, coupling of the aldehyde groups with 1, 3- diaminopropane to form Schiff base linkages and reduction of the Schiff's base linkages to form stable carbon-nitrogen bonds. In a typical procedure, 20 g of dextran were dissolved in 150 ml of 50 mM potassium acetate buffer, pH 6.5. A solution of 2.14 g of sodium peroiodate in 25 ml of distilled water was added dropwise to the dextran over about 10 minutes with vigorous magnetic mixing. The resulting solution was stirred at room temperature, 15-27°C, for about 1.5 hours and then dialyzed against distilled water. 20 ml of 1,3-diaminopropane were mixed with 20 ml of distilled water, cooled in an ice bath, vigorously stirred and pH adjusted from about 11.5 to about 8.7 over about 15 minutes by the addition of glacial acetic acid. Typically, 15-20 ml of glacial acetic acid was used. The dialyzed dextran solution was added dropwise over about 15-20 minutes to the chilled diamine solution. After the addition was completed, the resulting solution was stirred at room temperature for about 2.25 hours. A reducing solution of 0.8 g sodium borohydride in 10 ml of 0.1 mM sodium hydroxide was added to the dextran reaction mixture at room temperature over about 15 minutes. The reaction mixture was stirred during the borohydride addition to expel most of the effervescence. The crude aminodextran solution was exhaustively dialyzed against distilled water until the conductivity of the effluent was 3-4 μmho/cm. The dialyzed solution was then filtered through an 0.2 μm filter and freeze-dried over 24 hours in a model TDS-00030-A, Dura-Dry microprocessor controlled freeze-dryer (FTS Systems, Inc.) to produce 4.25 of flaky, pale yellow crystals in 21% yield.

Method B. Large scale preparation of aminodextran.

The procedure of Method A was modified for the large scale prepartion of aminodextran and for increasing the number of amino groups introduced into dextran. Hollow fiber membrane filtration replaces dialysis and a smaller diamine-periodate molar ratio was used to avoid further cleavage of the sugar polymer into lower molecular weight fragments. A hollow fiber cartridge (polysulfone, 3 ft$^3$ membrane surface area, 1 mm diameter fibers and 5,000 MW cut-off) was mounted vertically with an input power pump (two pump heads, maximum flow rate of about 4.56 liters/ minute with No. 18 Norprene® food grade tubing) delivering 15-20 psi which corresponds to 5-10 psi in the retentate line. The filtrate was collected at 50-100 ml/min. Washing was done using 20-30 liters of distilled water over about 6-8 hours. The specific conductance was reduced to about 3-4 $\mu$mho-cm$^{-1}$ and the pH was 6.0-6.5. The feed volume was maintained at 2 liters during desalting and then concentrated to 800 ml in the first washing of oxidized dextran and to 400 ml in the second washing of aminodextran.

In a standard scaled-up preparation, 80 g of dextran were transferred to 1 quart [liter] glass blender bowl containing 600 ml distilled water. The solid was blended for about 2-5 minutes at medium speed to dissolve all the dextran. 8.56 g of sodium periodate were dissolved in 100 ml of distilled water and the resulting solution was added dropwise to the dextran solution over about 10 minutes using vigorous magnetic stirring. After the addition was completed, the resulting mixture was stirred at room temperature for an additional 3 hours. The resulting viscous reaction mixture was then diluted to 2 liters with distilled water and desalted using a hollow fiber cartridge. The initial specific conductance was 1.5 mmho-cm$^{-1}$ or higher and the initial pH was 4.0. About 18-22 liters of distilled water were used to obtain solution having a final pH of 6.0-6.5. The final volume of washed, oxidized dextran solution was 800 ml.

To the washed, oxidized dextran solution, 80 ml of colorless, liquid 1,3-diaminopropane were slowly added over about 10 minutes at room temperature. The resulting mixture was then stirred at room temperature for an additional 3 hours. After the stirring was finished, 3.2 g of sodium borohydride dissolved in 40 ml of 1 mM aqueous sodium hydroxide were added to the room temperature aminodextran reaction mixture over about 5 minutes with magnetic stirring. After the completion of the sodium borohydride addition, the resulting mixture was stirred for an additional 1 hour and then desalted using a hollow fiber cartridge. The initial specific conductance was 5.0 mmho-cm$^{-1}$ or higher and the initial pH was about 12.0. About 20-25 liters of distilled water were needed to reduce the specific conductance to about 3-4 $\mu$mho-cm$^{-1}$ and the pH to 6.0-6.5. The final volume of aminodextran solution was 400 ml. This solution was passed through a 0.2 $\mu$m sterile cellulose acetate filter unit and then freeze-dried over 48 hours to obtain 48 grams of flaky, pale yellow crystals, a 52% yield.

Elemental analysis (C,H,N) were obtained for two samples of aminodextran prepared from dextran T-2M by the methods described above. The analyses are:

Sample 1. 20 g dextran scale, desalting by dialysis.
  Obsd.: C, 43.04; H, 6.60, N, 1.09;
    O (by difference), 49.27.
Sample 2. 80 g dextran scale, desalting by membrane filtration.
  Obsd.: C, 42.53; H, 6.52 N, 1.01;
    O (by difference), 49.94
Calculated for $C_{46}H_{79}NO_{37} \cdot 3H_2O$:
  C, 42.76; H, 6.63; N, 1.08; O, 49.53

The analyses for aminodextran in the two preparations were very similar, thus indicating that the same product was obtained whether desalting was done by dialysis or by membrane filtration. The empirical formula obtained for Sample 1, $C_{46}H_{84}NO_{40}$, is very similar to the formula $C_{46}H_{79}NO_{37} \cdot 3H_2O$ based on 29 units of glucose ($C_6H_{10}O_5$), 1 unit of fully diamine-substituted sugar ring ($C_{12}H_{28}N_4O_3$) and twelve units of water. Therefore, the degree of diamine substitution in dextran was 1/30 in Sample 1 in contrast to a theoretical value of 1/12 based on 100% periodate cleavage and diamine substitution. The empirical formula obtained for Sample 2, $C_{49}H_{90}NO_{43}$, is very similar to the formula $C_{49}H_{84}NO_{40} \cdot 3H_2O$ based on 31 units of glucose, 1 unit of fully diamine substituted sugar ring and twelve units of water. The degree of substituion in dextran by diamine was 1/32 for Sample 2.

In the preparation of aminodextran coated particles, similar results were obtained using aminodextrans having average molecular weights of 10,000, 40,000 and 2,000,000 (T-10, T-40 and T-2M) with 1X (1X= 3.3% substitution of sugar residues), 2X (6.6%) 3X (9.9%) and 5X (16.5%) molar amounts of amino groups. All the aminodextrans were initially prepared according to methods A and B using 2 and 3 times the amount of sodium periodate used in the 1X oxidation of dextran. The amount of 1,3-diaminopropane used for Schiff base formation was kept constant.

Modifications have been made to the Methods A and B of preparing aminodextrans which were originally disclosed in Serial NO. 07/827,347. These modifications involve the oxidation and cleavage of the dextran glucose rings with periodate anion, diamine addition and sodium borohydride reduction of the Schiff's base. The modifications have led to increased yield on the aminodextrans, particularly the 5X-aminodextran. Generally, the first modification was to use only a ten percent (10%) excess of diamine over the stoichiometric 2:1 diamine:periodate molar ratio previously disclosed. Second, the diamine addition reaction was conducted at a temperature in the range of about 5-10°C. Third, the diamine addition reaction was spectroscopically monitored in the near ultraviolet (UV) region for Schiff base formation. Schiff's base formation was deemed completed when successive spectral analyses indicated a plateau was reached. The reaction was then stopped. These modifications reduced aminolysis of the polymeric sugar groups into lower weight fragments and thus gave higher yield of product after purification and concentration by hollow fiber membrane filtration. The hollow fiber filtration was done using polysulfone cartridge of 3 ft$^2$ membrane surface area, 1 mm diameter fiber having a 5,000 molecular weight cut off. The cartridge was mounted vertically in an input power pump having two pump heads delivering 15-20 psi with a maximum flow rate of 4.56 liter/minute when using No. 18 Norprene® food grade tubing. With this configuration, the pressure in the retenate line was about 5-10 psi. The filtrate was collected at 50-100 ml/min. Washing was done using 20-30 liters of distilled water over about 6-8 hours. The following method for preparing 5X-aminodextran is given to illustrate the modified procedure which is applicable to the preparation of all aminodextrans.

Method C. Preparation of 5X-Aminodextran.

T-2M dextran (50g, 0.308 mol, obtained from Sigma or Pharmacia) was added to a 1-quart or 1-liter glass blender bowl containing 300 ml of distilled water. The mixture was blended at maximum speed until all the dextran dissolved, typically about 3-5 minutes. A solution of 26.75g (0.125 mol) of $NaIO_4$ in 300 ml distilled water was added to dextran solution over about a 10 minute period using vigorous magnetic stirring. After the periodate addition was completed, the reaction mixture was stirred at room temperature for about an additional three hours. After the three hours, the 600 ml reaction volume had an initial specific conductivity of 9.7 mmho-cm$^{-1}$ and an initial pH of 2.5. The reaction mixture was diluted to two liters with distilled water and desalted using the hollow fiber cartridge. Washing was done using 15-18 liter of distilled water to obtain 600 ml of washed, oxidized dextran solution having a specific conductance of 10 mmho-cm$^{-1}$ and pH of 6.7.

The solution of oxidized dextran was cooled to about 8°C using an ice bath and 23.2 ml (0.275 mol) of 1,3-diaminopropane was added over about 10 minutes to the oxidized dextran solution. The resulting reaction mixture was stirred and maintained at the ice bath temperature. The formation of the yellow Schiff's base was monitored ever 10-15 minutes by measuring the 335 nm near UV absorbance of an extracted smaple. In a typical experiment, the measurements at 335 nm using a lmm path length cell were:

Table 1.

| minutes | absorbance values |
|---------|-------------------|
| 0 | 0.100 |
| 5 | 2.063 |
| 15 | 2.975 |
| 30 | 3.692 |
| 45 | 3.901 |
| 60 | 4.103 |
| 75 | 3.784 |

After the absorbance had reached a plateau, 19.3 g (0.500 mol) of sodium borohydride in 19.3 ml of 1 mM aqueous potassium hydroxide were added to the reaction mixture over about 10 minutes at ambient temperature with magnetic stirring. After the sodium borohydride addition was completed, the reaction mixture was stirred at ambient temperature for about an additional two hours. After the stirring was completed, spectroscopic measurement at 335 nm using a 1 cm path length cell gave an absorbance value of 0.067 units which indicates that the Schiff's base compound had essentially disappeared. The reaction mixture, about 1000 ml volume, was then desalted using the hollow fiber cartridge. The initial specific conductance was 43 mmho-cm$^{-1}$ and the initial pH was 11.0. About 18-20 liters of distilled water were used as wash liquid to produce about 300 ml of 5X-aminodextran solution having a specific conductance of about 4-6 μmho-cm$^{-1}$ and a pH of 6.5-7.0. The 5X-aminodextran solution was filtered through a 0.2 μm cellulose nitrate filter and freeze-dried over 48 hours in a model TDS-00030-A, Dura-Dry® microprocessor-controlled freeze-dryer (FTS Systems, Inc.) to produce 24g (48% yield) of flaky, pale yellow crystals. Elemental analysis: C= 45.83%, H= 7.00%, N=4.49%, O (by difference) = 42.68%. Calculated analysis for $C_{12}H_{22}O_{8.25}N$: C=46.15%, H= 7.10%, N= 4.48%, O=42.26%.

The empirical formula based on actual analysis is $C_{12}H_{22}O_{8.3}N$, which is very similar to the formula $C_{12}H_{22}O_{8.25}N$ based on 6 units of glucose per one unit of fully diamine-substituted sugar ring ($C_{12}H_{28}N_4O_3$). Therefore, the degree of diamine substitution in dextran was 1/7 in contrast to a theoretical value of 1/2.5 based on 100% periodate cleavage and diamine substitution. Repeat experiments at 100g and 300g dextran scales produced a product having the same degree of substitution.

Preparation of Magnetite and Other Magnetic Particles in Gelatin Solution.

Metal ferrites ($MFe_2O_4$ where M = $Fe^{2+}$, $Mn^{2+}$, $Zn^{2+}$, $Co^{2+}$, $Ni^{2+}$ and $Ba^{2+}$) including magnetite can be prepared in situ in type B gelatin as described in I above. To further illustrate the invention, manganese ferrite was prepared as follows.

62.5 mmol (62.5 ml) of 1M $Fe_2SO_4$ solution, 100 mmol (50 ml) of 2M $KNO_3$ solution, 31.25 mmol (31.25 ml) of 1M $MnSO_4$ solution and 72.92 ml of distilled water were mixed together in a one liter Pyrex® bottle and purged with $N_2$ gas for 10 minutes (Solution A). Then, 166.6 mmol (33.33 ml) of 5M KOH was added to Solution A, swept with $N_2$ gas and sonicated at room temperature (18-27°C) for a time in the range of about 5-10 minutes to yield a smooth, dark green slurry of $Fe(OH)_2$ gel. After sonication, 250 ml of 2% type B, alkali cured, 225 Bloom bovine skin gelatin solution were added to the $Fe(OH)_2$ gel, mixed, swept with $N_2$ gas, capped tightly, sonicated for about 5-10 minutes and placed undisturbed in a 90°C oven for about 24 hours. After oven heating, the Pyrex® bottle and contents were removed from the oven and allowed to cool to room temperature. After cooling was completed, the suspension of brown particles in the bottle was mixed and decanted into two 250 ml tissue culture flasks. The fraction in each flask was washed about five times with 1% w/v type B gelatin or with 1% 1X aminodextran solution using magnetic separation between washings. After washing, the magnetic particles were recombined, suspended in sufficient 1% 1X-aminodextran solution to make 500 ml total volume and sonicated for about 0.5 hour. The particles were stored at refrigeration temperatures of about 5-10°C for a time of 8 hours to at least six months. Storage was done as a 2.5% w/v solids suspension in 1X-aminodextran solution containing 0.1% w/v sodium azide. Alternatively, the particles may be used immediately after preparation in type B gelatin and washing with 1X-aminodextran.

Preparation of 5X-Aminodextran Coated Magnetic Particles.

Method 1. A quantity of stored manganese ferrite particles prepared according to the procedure described above and of uniform 0.3 μm size and spherical shape were washed a plurality of times with distilled water, dried at 105°C and the w/v solids in suspension was determined. A sample which analyzed as having 0.56% w/v solids was used herein.

100 ml of 0.56% w/v solids, gelatin prepared, aminodextran-stored manganese ferrite particles were magnetically separated and resuspended in an equal volume of 2% w/v 5X-aminodextran solution. The 100 ml suspension of brown particles was sonicated for about 10 minutes and then mixed overnight (about 8-16 hours) in a 250 ml tissue culture flask on an orbital shaker. After the mixing was completed, the pH was adjusted to 10.0 with about 20 μL of 5M aqueous potassium hydroxide. The adsorbed 5X-aminodextran was crosslinked by the addition of 1.173 ml of 25% aqueous glutaraldehyde (3.11 mmol) to the pH adjusted particle suspension and mixing the resulting suspension for a time in the range of 1-4 hours, preferably one hour, using an orbital shaker. The crosslinked particles were magnetically separated, the supernatant measured and discarded, and, to block and stabilize unreacted aldehyde groups, the particles resuspended in a volume of 1% 5X- aminodextran solution equal to that of the discarded supernatant. Alternatively, a polyamine solution such as ethylenediamine or 1,3-diaminopropane solution may be used to block the unreacted aldehyde groups. The molar amount of polyamine is about ten times the amount of glutaraldehyde used. The resuspended particles were shaken and sonicated to disperse them, and the resulting suspension was mixed overnight using an orbital shaker. A 23.5 ml sample of 10 mg/ml sodium borohydride (6.22 mmol $NaBH_4$) in 1 mM aqueous potassium hydroxide was then added to the suspension and the resulting mixture orbitally mixed for an additional 0.5 hour. The particles were then magnetically separated and washed a plurality of times (minimum three) with 1X PBS and concentrated to 22.4 ml volume to yield a 2.5% w/v solids suspension.

Method 2. A 60.2 ml sample of 0.83% w/v solids manganese ferrite particles suspended in 1% w/v 1X-aminodextran solution containing 0.1% sodium azide was magnetically separated and washed a plurality of times with 20 ml portions of 0.2 M aqueous sodium chloride. After washing, the particles were resuspended in sufficient 3.75 mg/ml 5X-aminodextran in 0.2 M sodium chloride solution (about 20 ml) to give a 2.5% w/v solids suspension. Next, for each milliliter of the 2.5% w/v solids suspension, 9 μL of 10 mg/ml EDAC·HCl in 0.2 M sodium chloride solution was added to the suspension (180 μL total). The resulting mixture was orbitally shaken in a 50 ml tissue culture flask for a time in the range of about 12-16 hours. The particles were magnetically separated, washed a plurality of times with distilled water and resuspended in 1X PBS to yield 20 ml of 2.5% w/v solids suspension of magnetic particle having a coating formed by a condensation reaction between gelatin and the aminodextran. This condensation reaction occurs between aminodextran amine groups and carboxylate groups present on the gelatin. EDAC removes the water which is formed during the condensation reactions as a urea. The use of a crosslinking agent such as glutaraldehyde is not necessary. The resulting particles are defined as being equivalent to particles having a first gelatin layer, a second aminodextran layer and crosslinked by a chemical crosslinking agent.

Determination of Crosslinked Gelatin and 5X-Aminodextran Layer Thickness.

A. Gelatin Thickness.

A glutaraldehyde crosslinking solution was prepared by mixing 56 μL of 25% aqueous glutaraldehyde with 5 ml of 1mM aqueous potassium hydroxide. The particles from 5 ml of a 2.5% w/v solids suspension of manganese ferrite particles prepared in type B gelatin as described and suspended in 2% w/v, type A, 175 Bloom gelatin in 0.1 M phosphate buffer solution, pH 8.4, were magnetically separated and the supernatant liquid discarded. The separated particles were resuspended in 5 ml of the glutaraldehyde solution and mixed, preferably using a roller mixer, for about 30 minutes. The particles were again magnetically separated and washed three times with 5 ml of 1 mM aqueous potassium hydroxide before resupension in 5 ml of 1 mM aqueous potassium hydroxide.

Elemental analysis was performed using 5 ml of crosslinked, gelatin coated manganese ferrite particles that were washed fifteen times with distilled water and dried to constant weight at 110°C. Analytical results were: Mn = 19.05%, Fe = 49.49%, C = 0.54%, H < 0.5%, N < 0.5% and O (by difference) = 30.92%. The percentage of carbon by weight in gelatin can be obtained from its amino acid content (The Theory of the Photographic Process, 4th Ed., T.H. James, ed (Macmillan, New York 19767), Chapter 2, page 52. The type A gelatin used herein gives C = 50.46%, H = 6.765%, N = 18.27%, O = 24.29% and S = 0.21%. Using this information, the gelatin layer thickness can be calculated.

Using 1 g of gelatin coated ferrite particles, there will be 1 g x 0.0054/0.5406 = 0.01070 g gelatin and 0.9893 g ferrite. The particle volume of a 0.29 μm diameter manganese ferrite sphere is $1.277 \times 10^{-14}$ cm$^3$ and the number of particles in 0.9893 g manganese ferrite is $1.827 \times 10^{13}$. Consequently, the mass of gelatin per ferrite particle is $5.856 \times 10^{-16}$ g.

Assuming a density of gelatin coating of 0.02 g/cm$^3$ from the 2% w/v gelatin before crosslinking with glutaraldehyde, the volume of gelatin per particle is $2.92 \times 10^{-14}$ cm$^3$. As a result, the total volume, gelatin plus ferrite, per particle is $4.205 \times 10^{-14}$ cm$^3$ and the radius of a gelatin-ferrite sphere is $2.157 \times 10^{-5}$ cm (0.2157 μm). The thickness of the gelatin coating on the manganese ferrite sphere is thus 0.2157 μm - 0.145 μm (the mean radius of the ferrite sphere) = 0.0707 μm (71 nm). This is in good agreement with the values of 750 Angstroms on glass given A.T. Kudish et al., Proteins at Interfaces, ACS Symposium Series 343, J.L. Brash et al., eds. (American Chem. Soc., Washington, D.C. 1987), pages 261-277; 75 nm on mica given by N. Kawanishi, et al., J. Phys. Chem. 94: 4611-4617 (1990); and 600-700 Angstroms on glass given by H. Metzer et al., J. Colloid Interface Sci. 126: 292-303 (1988).

The gelatin layer thickness was similarly calculated for manganese ferrite particles where the gelatin layer was crosslinked using polyvinylpyrrolidone (PVP) stabilizer. Elemental analysis gave: Mn = 18.84%, Fe = 47.82%, C = 1.67%, H < 0.5, N < 0.5 and O (by difference) = 31.67. The calculated thickness of the gelatin coating was calculated as 148 nm. The thicker coating using PVP is believed to result from procedural differences. The thicker coating was obtained when the ferrite-gelatin solution is diluted, in the original procedure, from 2% to 0.08% and 1% PVP is added. The thinner coating was obtained when the gelatin-ferrite particles were separated from the 2% gelatin solution and resuspended in the crosslinking medium.

As a result, there was no excess polymer, either gelatin or PVP, present.

B. Aminodextran Thickness.

Manganese ferrite particles were coated with 5X-aminodextran (Method 1) which was subsequently crosslinked, blocked and stabilized. The resulting particles were washed a plurality of times with distilled water, magnetically separated and dried at 110°C. Elemental analysis results were: Mn = 14.04%, Fe = 44.36%, C = 2.97% and O (by difference) = 38.63%. For 5X-aminodextran analyzed as containing 45.83% C and a 2% w/v 5X-aminodextran coating, the estimated aminodextran layer has a thickness of 218 nm.

Activation of Diamine Treated Particles with Sulfo-SMCC.

The same procedures described for activating gelatin coated particles with sulfo-SMCC are used to activate the 5X-aminodextran coated particles except that five times the activating amount of sulfo-SMCC were used. When the magnetic particles are coated with 1X-, 2X- or 3X-aminodextran, one, two or three times the amount of sulfo-SMCC is used, respectively.

Antibody Activation with 2-Iminothiolane Hydrochloride.

Antibodies were activated with 2-iminothiolane hydrochloride according to the procedures previously described herein. If the antibody or other substance which is to be conjugated to the activated particles has a sufficiency of reactive sulfhydryl group, activation by 2-iminothiolane hydrochloride may not be necessary. Those skilled in the art will also recognize that the activation of particles to contain maleimidyl groups and the activation of antibodies or other substances can be switched. That is, the particles can be activated by the introduction of reactive sulfhydryl groups and the antibody or other substance can be activated to contain reactive maleimidyl groups.

The following example is given to illustrate the utilitiy of the invention and are not to be taken as limiting the invention. Unless otherwise indicated, Method 1 was used to coat particles with 5X-aminodextran. In this example, two methods for enumerating neutrophils in a leukocyte-rich (leuko-rich) whole blood sample are described. The monoclonal antibody 1D3 conjugated to 5X-aminodextran coated beads and flow cytometry is used in both methods.

In the first method, a series of 1D3 containing magnetic bead titers were prepared, mixed with the leuko-rich blood sample and then magnetically separated from the sample. The neutrophil-depleted supernatants were then analyzed by flow cytometry to determine the point at which neutrophil depletion was complete. Samples were analyzed in the order of lowest titer to highest titer and depletion was recognized by a constant cytometer count.

In the second method, whole blood samples were mixed with the same titers of 1D3-conjugated magnetic beads, but no magnetic separation was performed. The mixtures were analyzed by flow cytometry for neutrophils shifted out of the normal granulocyte region in the forward verus side (orthogonal) scatter histogram because of a change in cell size, shape or refractive index due to attachment of magnetic beads to the neutrophil cell surfaces. When the shifted neutrophil population or count reached a plateau value, its count in the light scatter histogram or its fluorescent event count in the bitmapped region was compared to the respective counts for all white blood cells (lymphocytes, monocytes and granulocytes) to obtain the relative number of neutrophils.

The use of well-defined ferrite particles of uniform size, uniform spherical shape and uniform refractive index properties is essential in obtaining a recognizable, definitive shift in light scatter from biological cells conjugated to the surface of particles such as the particles described herein. In addition to electric dipole scattering arising from chemical substances, magnetic dipole scattering from the ferrite particles can make a substantial contribution to the intensity of the light scattering arising from the cell-particle conjugates [J.A. Stratton, Electromagnetic Theory (McGraw Hill, New York 1941), page 437, and C.F. Bohren and D.R. Huffman, Adsorption and Scattering of Light by Small Particles (Wiley, New York 1983), page 141]. J.J. Murry, Optics 4: 1011 (1965), has measured low angle light scattering by magnetic particles such as magnetite.

The diameter of the manganese ferrite particles which are used herein was $0.29 \pm 0.08$ μm. This diameter lies outside the mean diameter range of 0.65-3.0 μm for the polystyrene latex and magnetic latex particles which were used in International Patent Application Publication WO 90/13013 entitled METHOD AND APPARATUS FOR SCREENING CELLS OR FORMED BODIES WITH POPULATIONS EXPRESSING SELECTED CHARACTERISTICS UTILIZING AT LEAST ONE SENSING PARAMETER and WO9209682 (to Coulter Corporation, the assignee herein) entitled METHOD AND APPARATUS FOR SCREENING MICROSCOPIC CELLS UTILIZING LIGHT SCATTER TECHNIQUES. The polystyrene latex particles used in these studies were of uniform size and shape. However, while the magnetic latex particles, which were formed by embedding ferrofluid particles of varying size and shape inside the latex during emulsion polymerization (U.S. Patent No. 4,358,388 to Daniel et al.), were spherical in shape, they varied widely in size. For example, while the mean particle diameter was 0.7 μm, individual particles varied from 0.2 μm to 1.0 μm. (An additional problem arises from the fact that the structure of the ferrofluid particles embedded in polystyrene latex is not definable.) The small diameter particles, though low when measured by weight percent, were very numerous. Consequently, due to the higher mobility of the smaller particles relative to the mobility of the larger particles, the smaller particle-antibody conjugates will preferentially occupy antigenic sites on cell surfaces. However, the contribution of these small particles toward shifting the light scatter of cells is minimal and the use of these magnetic particles to obtain a true enumeration or count of targeted shifted cell populations is unreliable. This may, in part, be due to a resultant low net magnetic dipole when a summation is carried over many magnetic particles of different special orientation within the latex particles.

In addition to their uniform size and uniform spherical shape, the index of refraction of dense, microcrystalline ferrite particles that are formed and coated as described here is sufficiently different from the index of refraction of polystyrene latex and biological cells. When selected cell sets or subsets are coupled to these magnetic particles, large shifts occur in the forward versus side scatter histograms of the conjugated cells relative to the histogram of unconjugated cells. Consequently, cells conjugated to the particles can be distinguished from those not conjugated to particles.

Example 1 Enumeration of Neutrophils.

Preparation of Samples.

A 50 ml sample of $Na_4EDTA$-anticoagulated whole blood was divided among a plurality of centrifuge tubes and centrifuged at 500g for about 10 minutes. The majority of the plasma was removed and the buff colored layer of cells in each tube was removed, pooled and centrifuged again at 500g for about 10 minutes. Collectively, the buff colored cells and the plasma constitute the leuko-rich whole blood which should have a red blood cell (rbc) count no greater than $8.0 \times 10^8$/ml and a white blood cell (wbc) count between $2-4 \times 10^7$ /ml.

100 µL of the leuko-rich buff colored cells were pipetted into a plurality of reaction tubes. A quantity of 5 to 250 µL of 2.5% w/v suspended solids, 1D3-conjugated, 5X-aminodextran coated manganese ferrite particles prepared as described herein was pipetted into separate tubes. Typically the titers of ferrite particle suspension were 0, 5, 10, 20, 30, 40, 50, 75, 100, 150, 200 and 250 µL. The volume of each tube was then made up to 350 µL by the addition of 1X PBS. Each tube was nutated for 6 minutes at low speed and the contents of each tube were magnetically gathered and separated for 60 seconds. The supernatant liquid which constitutes a leukocyte-depleted (leuko-depleted) sample was carefully removed by pipette and placed in duplicate tube for analysis.

Leuko-rich (undepleted) and leuko-depleted buff colored cell samples were flow cytometrically analyzed to discriminate between actual neutrophil depeletion and non-specific neutrophil depletion; that is, depletion of lymphocytes, monocytes, eosinophils and basophils in addition to neutrophils. Each sample undergoing analysis was treated by the addition of 10 µl of the single color antibody reagent CYTO-STAT® KC56-FITC (Coulter Corporation, Miami, Florida), vortexed and incubated for 10 minutes at room temperature. The control samples were isotype control and antibody control conjugated to undepleted buff colored cells. The tubes were then placed in a Counter EPICS® Q-prep or similar instrument and processed on the 35 second lyse mode. After the rbcs were lysed and the samples fixed (Q-prep), the total volume of each sample was 1,100 µL. All samples were then analyzed on a Coulter EPICS® Profile II flow cytometer or similar instrument. The programs available on the Profile II instrument were used to analyze lymphocyte and monocyte-myeloid populations as fluorescent events. To estimate the number of cells per volume of sample, the wbc count of the undepleted sample was determined on a Coulter S-Plus® counter ($4.19 \times 10^6$ wbc), 100 µL sample aspiration, and was compared with the total fluoresecent events measured ($1.81 \times 10^6$) for lymphocytes, monocytes and granulocytes in the undepleted sample and 100 µL sample aspiration. As a result of these measurements, the fluorescent events determined for the depleted samples were scaled by a factor of 2.31 [($4.19 \times 10^6$) ÷ ($1.81 \times 10^6$)] and a dilution factor of 11 to obtain actual cell counts.

Procedure 1. Flow Cytometry Analysis for Neutrophils After Magnetic Bead Depletion of Samples.

A myeloid cell assay of an undepleted control sample resulted in 29,131 lymphocyte, 19,282 monocyte and 116,479 granulocyte counts as fluorescent events. The granulocytye percentage in the total wbc population is therefore 70.6%. Results obtained using the Coulter S-Plus® counter gave a granulocyte total of 71.7%. Depletion analysis was done using 5, 10, 20, 30, 40, 50, 75, 100, 150, 200 and 250 µL of 0.25% w/v solids magnetic beads conjugated with 1D3 monoclonal antibody. The results (Table 2) were:

Table 2.

| µL Beads | Counts | | |
|---|---|---|---|
| | lymphocytes | monocytes | granulocytes |
| 5 | 32,428 | 11,638 | 106,421 |
| 10 | 32,243 | 9,971 | 86,017 |
| 20 | 32,200 | 5,477 | 42,296 |
| 30 | 33,884 | 4,178 | 31,505 |
| 40 | 30,788 | 2,166 | 23,008 |
| 50 | 30,707 | 1,597 | 16,117 |
| 75 | 29,914 | 1,373 | 13,471 |
| 100 | 28,327 | 961 | 10,645 |
| 150 | 24,427 | 701 | 8,127 |
| 200 | 21,127 | 512 | 7,147 |
| 250 | 16,933 | 470 | 6,397 |

Both neutrophils and monocytes were depleted throughout the titer with 1D3-conjugated magnetic beads. Monocyte removal can be avoided or substantially reduced by shortening the nutation time for the buff cell and magnetic bead mixture from 6 minutes to 30 seconds and reducing the magnetic separation time from 60 seconds to 15 seconds. Based on the difference between undepleted and depleted sample fluorescent events for granulocytes and the total wbc fluorescent events in the undepleted sample, the percentage of neutrophils in the total wbcs was 66.8%.

The neutrophil depletion data were converted to bead and cell values using a value of $4.18 \times 10^{10}$ manganese ferrite particles/ml of 0.25% w/v solids suspension and the factor 25.41 (the fluorescent event scale factor 2.31 times the dilution factor 11, both obtained above) to convert fluorescent events to granulocyte cell counts. These resulting values (Table 3) are:

Table 3.

| μL Beads | No. Magnetic Beads x $10^8$ | No. Neutrophils Depleted x $10^6$ | Bead-to-Cell Ratio x $10^2$ |
|---|---|---|---|
| 5 | 2.1 | 0.26 | 8.1 |
| 10 | 4.2 | 0.77 | 5.5 |
| 20 | 8.4 | 1.9 | 4.4 |
| 30 | 12.6 | 2.2 | 5.7 |
| 40 | 16.8 | 2.4 | 7.0 |
| 50 | 21 | 2.6 | 8.1 |
| 75 | 31.5 | 2.6 | 12.1 |
| 100 | 42 | 2.7 | 15.6 |
| 150 | 63 | 2.8 | 22.5 |
| 200 | 84 | 2.8 | 30 |
| 250 | 105 | 2.8 | 37.5 |

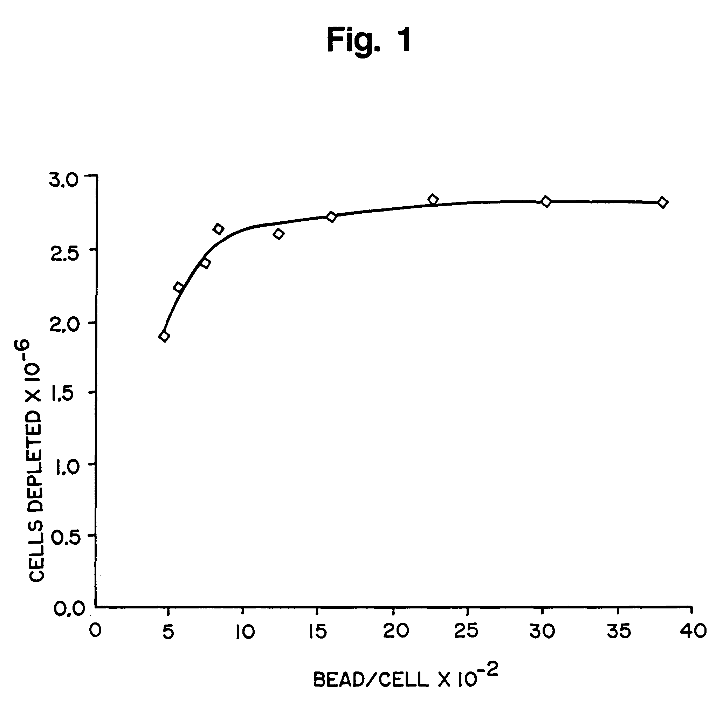

Fig. 1, which graphically depicts the number of neutrophils depleted versus the magnetic bead-to-cell ratio, shows that a plateau in neutrophil depletion was reached between the 100 μL and 150 μL 0.25% w/v magnetic bead titers when 2.8 x $10^6$ neutrophils were completely depleted using a bead-to-cell ratio of about 2000:1. This represents 66.8% of the total wbc count of 4.19 x $10^6$ determined by the Coulter S-Plus® counter.

Procedure 2. Flow Cytometry Analysis for Neutrophils Shifted by Magnetic Beads.

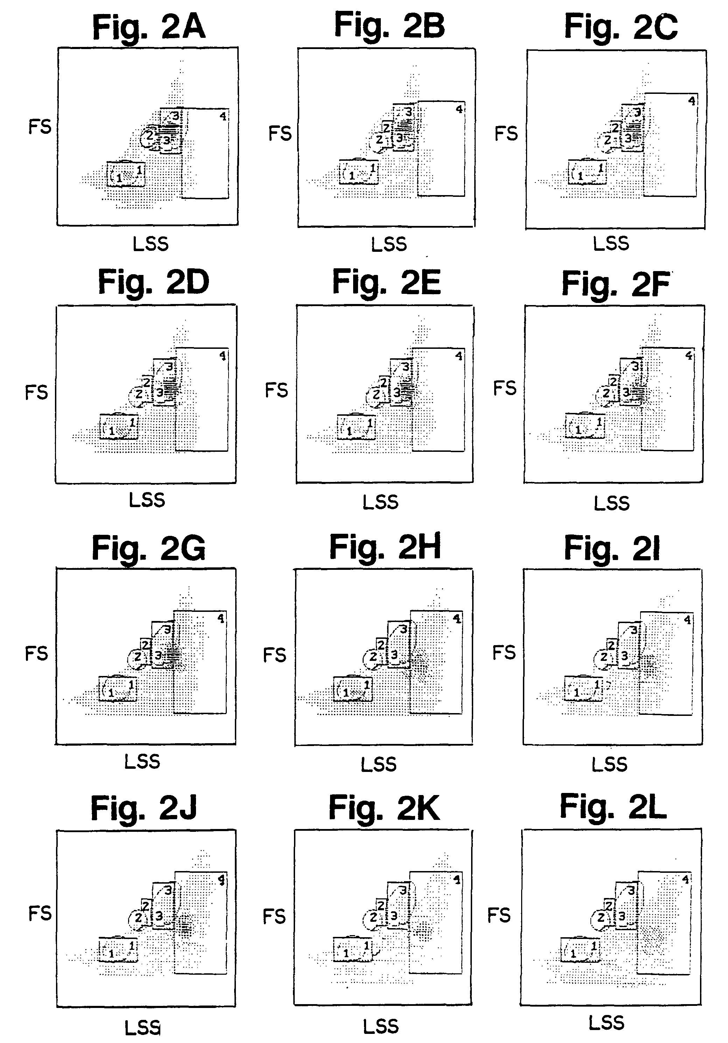

Leuko-rich buff cell-magnetic bead mixtures were prepared as described in the Preparation of Samples for analysis using a Coulter Profile II cytometer, but no magnetic separation was done. Programs were introduced into the profile to analyze lymphocyte and monocyte-myeloid populations in the forward versus side scatter histogram; in particular, those neutrophils shifted out of the normal granulocyte region due to a change in size, granularity or refractive index brought about by binding the neutrophils to the magnetic ferrite particles. The series of histograms in Fig. 2 show a distinct and progressive shift toward less forward scatter (FS) or smaller size and more side scatter (LSS) or greater granularity in the normal granulocyte region as higher titers of magnetic beads were added to the cell samples.

The refractive index and magnetic properties of ferrite particles can, however, upset the usual trend to larger size (greater FS) and greater granularity (larger LSS) expected as particles are attached to targeted cells. Monocytes were shifted in the same direction as the granulocytes with the end result that at the highest magnetic bead titers they occupied the region normally occupied by granulocytes. Lymphocytes remained unshifted until titers greater than 100 μL of 0.25% solids magnetic beads were used. At these higher titers, lymphocytes were apparently removed because there is a shift out of the lymphocyte boxed region of the forward versus side scatter histogram. This apparent shift occurs when there is present an excess of magnetic beads not bound to either monocytes or neutrophils. The excess beads appear to disturb the granularity of lymphocytes not by binding them, but by back-scatter from the excess magnetic ferrite particles. The following Table 4 gives the original granulocyte count, the shifted granulocyte count and the percent of granulocytes shifted using different titer of 0.25% w/v solids, 1D3 conjugated, 5X-aminodextran coated manganese ferrite particles.

Table 4.

| μL Beads | Granulocyte | | |
|---|---|---|---|
| | Original Count | Shifted Count | % Shifted |
| 0 | 120,977 | 10,413 | 0 |
| 5 | 121,892 | 16,537 | 11.9 |
| 10 | 115,195 | 31,918 | 21.7 |
| 20 | 87,148 | 55,773 | 39.0 |
| 30 | 84,669 | 60,156 | 41.6 |
| 40 | 73,559 | 74,978 | 50.5 |
| 50 | 54,482 | 72,032 | 56.9 |
| 75 | 16,987 | 80,129 | 82.5 |

Table 4.  (continued)

| μL Beads | Granulocyte | | |
|---|---|---|---|
| | Original Count | Shifted Count | % Shifted |
| 100 | 17,449 | 88,145 | 83.5 |
| 150 | 14,766 | 87,669 | 85.6 |
| 200 | 9,720 | 84,772 | 89.7 |
| 250 | 7,396 | 76,362 | 91.2 |

For the 100 μL titer, the total analysis results were 26,604 lymphocytes counts, 1,045 monocytes counts 17,449 unshifted granulocytes counts and 88,145 shifted granulocyte counts. As a result, the percent neutrophils in the total wbc count was calculated as 66.2%. The neutrophil percentage obtained by the population shift method agrees well with the 66.8% calculated from the magnetic depletion data obtained using Procedure 1.

To further illustrate the utility of the invention, additional depletion studies where performed on (a) red blood cells and platelets, and (b) white blood cells.

Example 2. Magnetic Bead Depletion of Red Blood Cells and Platelets.

100 μL of $Na_4$ EDTA-anticogulated whole blood were placed in a plurality of reaction tubes. Titers of 20-160 μL of 2.5% w/v solids, KC-16 conjugated, SX-aminodextran coated manganese ferrite particles were added to the reaction tubes and the total volume in each tube was adjusted to 260 μL with 1X PBS. The resulting mixtures were nutated for 6 minutes in either a multi-tube vortexer or a single tube nutator at low mixing speed. When nutation was completed, the beads in each tube were magnetically separated for 60 seconds. The supernatant liquid was removed using a Pasteur pipette and was saved in labelled tubes. The samples were analyzed using a Coulter S-Plus® or similar rbc counter as the total rbc count per 100 μL of sample (whole blood plus beads plus 1X PBS). The positive control was 100 μL whole blood plus 160 μL 1X PBS to give 100% rbc count. The percentage rbcs depleted = 100% -[rbc count in sample tube) ÷ (100% rbc count)].

The following Table 5 summarizes the results of an erythrocyte/thrombocyte assay. The 0 μL data is the undepleted control sample. The depletion was done using 1.25% w/v solids, KC-16 conjugated, 5X-aminodextran coated beads. KC-16 monoclonal antibody binds only to erythrocytes and does not bind leukocytes or platelets.

Table 5.

| μL Beads | Counts | | |
|---|---|---|---|
| | wbc x $10^5$ | rbc x $10^8$ | platelets x $10^6$ |
| 0 | 2.1 | 1.71 | 7.2 |
| 20 | 2.1 | 1.34 | 7.1 |
| 40 | 2.1 | 0.89 | 7.1 |
| 60 | 2.3 | 0.54 | 7.8 |
| 80 | 2.4 | 0.36 | 8.1 |
| 100 | 2.4 | 0.25 | 8.0 |
| 120 | 2.5 | 0.15 | 8.5 |
| 140 | 2.3 | 0.04 | 8.5 |
| 160 | 2.3 | 0.03 | 8.3 |

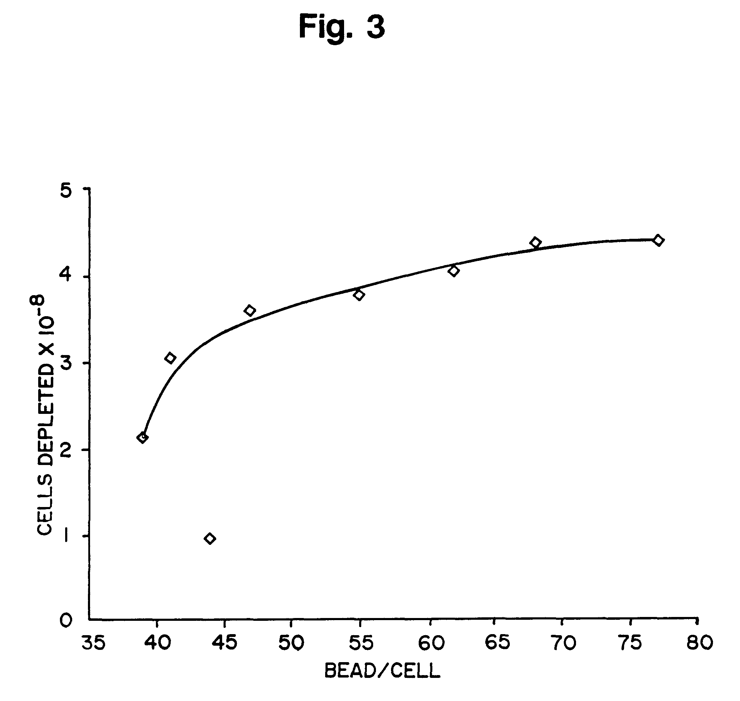

The number of magnetic beads/ml for a 2.5% w/v solids suspensions is 4.18 x $10^{11}$. Using Fig. 3, a plot of the number of rbcs depleted versus bead-to-cell ratio, 160 μL of 1.25% w/v solid beads contained 3.36 x $10^{10}$ magnetic particles and removed 4.37 x $10^8$ rbcs, thus giving a particle-to-(bound rbc) ratio of 77.

The assay was repeated for magnetic beads coated with 5X-aminodextran by Method 2. The assay was performed using 5, 10, 20, 40, 60, 80, 100, 150, 200 and 250 μL titers of 2.5% w/v solid, KC-16 conjugated, 5X-aminodextran coated manganese ferrite particles. The positive control was 100 μL of whole blood in 250 μL 1X PBS which was subsequently diluted to 1.000 ml with 1X PBS. The wbc, rbc and platelet counts for the undepleted control are listed in the following depletion Table 6 as 0 μL beads.

Table 6.

| µL Beads | Counts | | |
|---|---|---|---|
| | wbc x $10^4$ | rbc x $10^7$ | platelets x $10^6$ |
| 0 | 8 | 4.7 | 1.8 |
| 5 | 8 | 4.5 | 1.9 |
| 10 | 8 | 4.4 | 1.8 |
| 20 | 8 | 37 | 1.8 |
| 40 | 8 | 2.3 | 1.7 |
| 60 | 7 | 1.5 | 1.8 |
| 80 | 7 | 0.9 | 2.1 |
| 100 | 6 | 0.6 | 1.8 |
| 150 | 6 | 0.4 | 1.8 |
| 200 | 6 | 0.2 | 1.7 |
| 250 | 6 | 0.1 | 1.8 |

A plot of number of rbcs depleted versus particle-to-cell ratio will have a plateau similar to that shown in Fig. 2. 150 µL of 2.5 w/v solids magnetic particles, at the plateau, contained 6.3 x $10^{10}$ particles and removed 4.3 x $10^8$ rbcs for a particle-to-rbc ratio of 147.

A depletion assay for platelets was performed and 10, 20, 30, 40, 50, 100, 150 and 200 µL of 0.83% w/v solids, PLT-1 monoclonal antibody (Coulter Corporation, Hialeah, Florida) conjugated, 5X-aminodextran coated manganese ferrite particles. Total volume was adjusted to 350 µL by the addition of 1X PBS as needed. The positive control was 100 µL of whole blood diluted with 250 µL of 1X PBS. The wbc, rbc and platelets for the undepleted control are listed in the following depletion Table 7 as 0 µL beads.

Table 7.

| µL Beads | Counts | | |
|---|---|---|---|
| | wbc x $10^5$ | rbc x $10^8$ | platelets x $10^6$ |
| 0 | 1.7 | 1.51 | 5.2 |
| 10 | 1.6 | 1.48 | 3.1 |
| 20 | 1.6 | 1.51 | 1.2 |
| 30 | 1.5 | 1.50 | 0.4 |
| 40 | 1.4 | 1.55 | 0.3 |
| 50 | 1.3 | 1.45 | 0.2 |
| 100 | 1.1 | 1.57 | 0.1 |
| 150 | 1.1 | 1.46 | 0.1 |
| 200 | 1.0 | 1.49 | 0.1 |

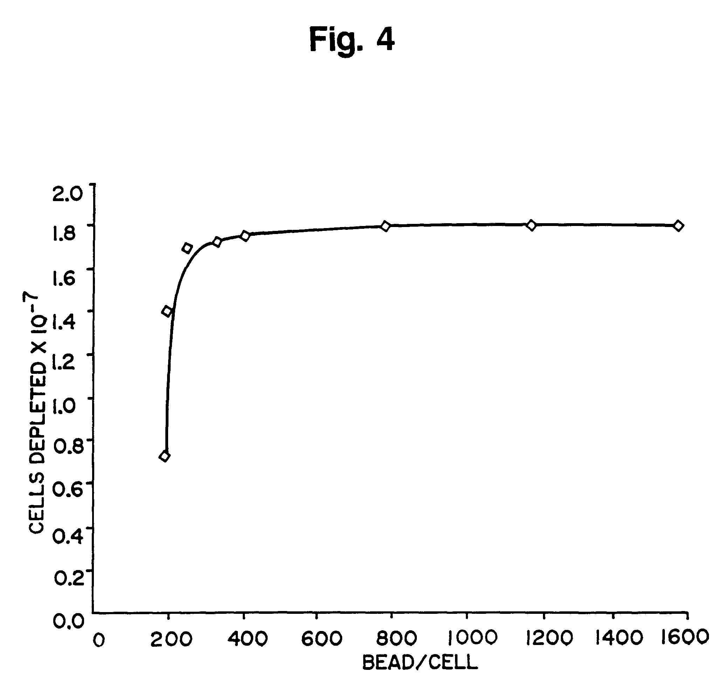

The results as plotted in Fig. 4 indicate that 100 µL of 0.83% w/v solids beads containing 1.39 x $10^{10}$ particles removed 1.79 x $10^7$ platelets, yielding a particle-to-platelet ratio of 779.

Example 3. Magnetic Bead Depletion of White Blood Cells.

The preparation of leuko-rich samples, their depletion with KC-56 monoclonal antibody (Coulter Corporation) conjugated, 5X-aminodextran coated manganese ferrite particles and the analysis of the supernatant liquids after magnetic depletion according to Procedure 1 are similar to those described for neutrophil depletion using 1D3 antibody-conjugated magnetic beads. The depletion assay was conducted using 5, 10, 20, 40, 60, 80, 100, 150, 200 and 250 µL of 0.833% w/v solids, KC-56 conjugated, 5X-aminodextran coated manganese ferrite particles. The lymphocyte, monocyte and granulocyte counts for the undepleted control sample, given as fluorescent events, are listed in the following depletion Table 8 as 0 µL beads.

Table 8.

| µL Beads | Counts | | |
|---|---|---|---|
| | lymphocytes | monocytes | granulocytes |
| 0 | 25,461 | 11,730 | 40,740 |
| 5 | 15,660 | 6,447 | 53,165 |
| 10 | 13,746 | 5,801 | 60,708 |
| 20 | 3,474 | 3,589 | 50,268 |
| 40 | 1,266 | 1,362 | 30,370 |
| 60 | 251 | 123 | 6,147 |
| 80 | 100 | 83 | 4,333 |
| 100 | 89 | 5 | 201 |
| 150 | 16 | 0 | 8 |
| 200 | 6 | 1 | 4 |
| 250 | 0 | 0 | 1 |

To determine the number of cells per volume of sample, the wbc count ($2.86 \times 10^6$) of the undepleted sample was determined for a 100 µL aspiration on a Coulter S-Plus® counter and this count compared with the total fluorescent events ($8.57 \times 10^5$) for lymphocytes, monocytes and granulocytes in an undepleted sample and 100 µL aspiration on the Coulter EPICS® Profile II flow cytometer. As a result, the fluorescent events were scaled by a factor of 3.34 [that is, ($2.86 \times 10^6$) ÷ ($8.57 \times 10^5$)] and the dilution factor of 11 to obtain cell numbers. Bead numbers were calculated using $1.39 \times 10^{11}$ particles/ml for a 0.833% w/v solids suspension. The following depletion Table 9 tabulates the results as number of wbc depleted x $10^5$, number of magnetic beads using in the depletion x $10^8$ and the bead-to-cell ratio x $10^3$.

Table 9.

| µL Beads | WBC x $10^5$ Depleted | No. Beads x $10^8$ Used in Depletion | Bead-to-Cell Ratio x $10^3$ |
|---|---|---|---|
| 0 | | | |
| 5 | 5.54 | 6.97 | 1.26 |
| 10 | 6.48 | 13.9 | 2.15 |
| 20 | 11.1 | 27.9 | 2.52 |
| 40 | 16.5 | 55.8 | 3.38 |
| 60 | 26.2 | 83.6 | 3.19 |
| 80 | 27.0 | 111 | 4.13 |
| 100 | 28.5 | 139 | 4.89 |
| 150 | 28.6 | 209 | 7.30 |
| 200 | 28.3 | 279 | 9.74 |
| 250 | 28.6 | 348 | 12.2 |

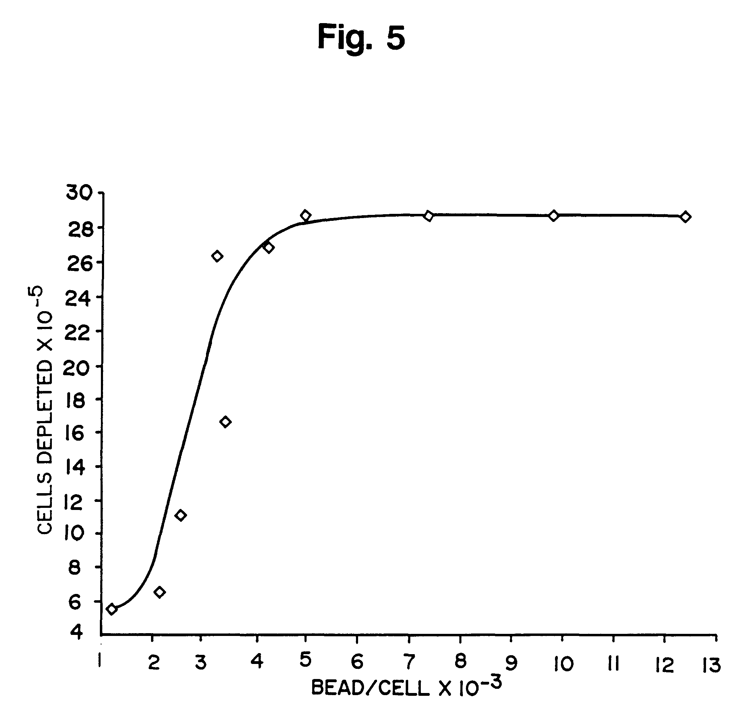

Fig. 5 is a plot of the number of wbcs depleted versus the magnetic bead-to-cell ratio. Fig. 5 shows a steep rise for the bead-to-cell values of $2.15 \times 10^3$ and $4.13 \times 10^3$, and a plateau starting at 100 µL of 0.833% w/v bead titer where $2.85 \times 10^6$ wbcs were depleted at a bead-to-cell ratio of about 4900:1. The removal of the larger granulocytes cells begins after almost all of the lymphocytes and monocytes were depleted. This is consistent with the requirement of needing a greater number of magnetic beads attached to a granulocyte to magnetically remove these cells. That is, for magnetic separation of granulocytes it is necessary to have more magnetic particles attached to each of the larger granulocyte cells in order to have sufficient magnetic moment to physically pull the cells from solution toward the magnetic field. There is no separate inflection point shown in Fig. 5 for granulocyte depletion. However, some granulocytes were shifted in the forward versus side scatter histograms for higher titers and just prior to complete depletion. This indicates the presence of some granulocytes that were not removed by magnetic separation, but remained in suspension with significant numbers of magnetic beads attached to the cells.

III. PREPARATION OF POLYMERIC PARTICLE WITH BIODEGRADABLE COATINGS

A. GELATIN COATED POLYMERIC PARTICLES (for Reference only; not an example of the Invention)

Preparation of Gelatin Coated Polystryrene Latex Particles.

Sulfated polystyrene latex particles (IDC Corporation. Portland, Oregon) of uniform size (2.17 μL ±3.0%) and spherical shape were used to exemplify the invention and were dispersed in distilled water and centrifuged for 10 minutes at 3000 rpm. The supernatant liquid was discarded and the particles were resuspended in 1% aqueous, type A, 175 Bloom gelatin at 2.5% w/v solids, sonically mixed for 1 minute to aid redispersion and roller mixed for 8-16 hours. The sulfated polystyrene latex particles did not have amine-reactive groups attached to the polystryene. However, groups can be used in the same manner even though gelatin contains few, if any, amine groups. Furthermore, the polystyrene latex particles may have embedded magnetic particles or the particle may consist of a magnetic nucleus having a polymer coating, in this case a coating of polystyrene latex. If such magnetic particles are used, then separations may be done magnetically instead of by centrifugation.

Crosslinking the Adsorbed Gelatin and Blocking Unreacted Aldehyde Groups.

A 0.300 ml aliquot of 25% aqueous glutaraldehyde (0.749 mmol) was added to 575 ml phosphate buffered saline (1X PBS) containing 1% polyvinylpyrrolidone (40,000 MW). 25 ml of 2.5% w/v solids sulfate polystyrene latex particles in 1% type A, 175 Bloom gelatin solution were then added to the glutaraldehyde solution. The resulting suspension was placed in a 1L polypropylene centrifuge bottle and roller mixed for 6 minutes. After mixing, 0.505 ml of 99% ethylenediamine (7.49 mmol) was added to the 600 ml of particles in 1X PBS and the resulting suspension was roller mixed for about 2-3 hours. 6.0 ml of 10 mg/ml NaBH$_4$ in 0.1 mM aqueous KOH were added and the suspension again roller mixed for 15 minutes. The particles were washed three times with 0.2M aqueous NaCl by centrifugation and decantation. After washing, the particles were resuspended in 0.2M NaCl to yield 24 ml of 2.5% w/v solids suspension.

Crosslinking the Adsorbed Gelatin Without Polyvinylpyrrolidone Stabilizer.

56 μL of 25% aqueous glutaraldehyde solution were mixed with 5 ml of 1 mM aqueous potassium hydroxide. 5 ml of 2.5% w/v solids sulfate polystryrene latex particles suspended in 2% w/v, type a gelatin, 0.1 M in phosphate buffer, pH 8.4, were separated by centrifugation and the supernatant liquid was discarded. The separated gelatin coated polystryrene latex particles were suspended in 5 ml of the 3 mg/ml glutaraldehyde solution prepared as described herein. The resulting suspension was miexed, preferably roller mixed, for about thiry minutes. After the glutaraldehyde addition and mixing was completed, 20 μL of ethylenediamine (2:1 diamine-to-glutaraldehyde molar ratio) was added to the reaction mixture which was then stirred for an additional 2-3 hours. Subsequently, 0.313 ml of a 40 mg/ml solution of sodium borohydride (100 mg per gram of beads) in 1 mM KOH was added to the reaction and the resulting mixture stirred for about 10-30 minutes. The crosslinked particles wer ethen separated by centrifugation and washed three times, and resuspended in 5 ml of 0.2 M aqueous sodium chloride.

Coupling of Ethylenediamine to the Carboxylate Residues of Gelatin Coated on Polystyrene Latex Particles.

A. 0.404 ml aliquot of 99% ethylenediamine (6.00 mmol) was mixed with 24 ml of fixed, aldehyde blocked, gelatin-coated polystyrene particles, 2.5% w/v solids, prepared with or without the use of polyvinylpyrrolidone stabilizer. A 0.960 ml sample of 1 mg/ml EDAC (0.50 mmol) in 0.2M NaCl solution was added to the particles and roller mixed for 8-16 hours in a 50 ml centrifuge tube. The contents of the tube were washed five times with 1X PBS after centrifugation for 10 minutes at 3000 rpm and decantation. The particles were then resuspended in sufficient 1X PBS to give a total volume of 24ml.

Particle Activation by Sulfo-SMCC and Antibody Coupling to the Gelatin Coated Polystyrene Latex Particles.

The activation of the particles and the coupling of monoclonal antibodies to gelatin coated polystyrene particles was carried out using the same procedures as followed for magnetic beads, except that separation of the particles was accomplished by centrifugation for 10 minutes at 3000 rpm followed by decantation of the supernatant liquid.

B. AMINODEXTRAN COATED PARTICLES.

Preparation of Aminodextrans.

The aminodextrans used to coat the polymeric particle were prepared as described above.

Preparation of Aminodextran Coated, Functionalized-Polystyrene Latex Particles.

Two types of functionalized polystyrene latex particles or beads were coated according to the invention using 5X-aminodextran as the exemplary coating material. The first type were Polybead carboxylate microspheres of 2.01 $\mu$m diameter (0.046 standard deviation) from Polysciences, Inc., Warrington, Pennsylvania, and the second type was aldehyde or aldehyde/sulfate polystyrene latex particles of 2.15 $\mu$m $\pm$ 5.5% diameter from IDC Corporation, Portland, Oregon. Neither of these particle types were magnetic. However, magnetic beads can be coated in the same manner and magnetic separation used instead of centrifugation to separate the beads from the suspending medium. The first step in coating the beads with 5X-aminodextran was to couple the 5X-aminodextran to particles surface functional groups by a condensation reaction between surface carboxylate or aldehyde groups and the amine groups of 5X-aminodextran.

Procedure 1. Coupling to Carboxylate Polystyrene Latex Particles.

Five milliliter (5 ml) of 10 mg/ml aqueous 5X-aminodextran solution and 1 ml of 2M aqueous NaCl were added to 4 ml of aqueous suspension of 2.5% w/v solids carboxylated polystyrene latex particles. 230 $\mu$L of 10mg/ml aqueous solution of a water soluble carbodiimide, for example, EDAC·HCl, was then added to the colloidal dispersion of latex particles and the entire mixture was mixed, preferably by means of a roller mixer, for a time in the range of about 8-16 hours.

Procedure 2. Coupling to Aldehyde/Sulfate Polystyrene Latex Particles.

Five milliliters (5 ml) of 10 mg/ml aqueous 5X-aminodextran and 2.619 ml of distilled water were added to 2.381 ml of aqueous suspension of 4.2% w/v solids aldehyde/sulfate polystyrene latex particles. The pH of the dispersion was then adjusted to 10.0 by the addition of 2 $\mu$L of 5M aqueous potassium hydroxide solution. The resulting mixture was then mixed, preferably by means of a roller mixer for a time in the range of about 8-16 hours.

Crosslinking of 5X-Aminodextran on Polystyrene Latex Particles.

The aminodextran coated beads prepared by either Procedure 1 or 2 were washed three to four times by suspension in distilled water and centrifugation for ten (10) minutes at 3000 rpm. The beads were then resuspended in 10 ml of 2% w/v aqueous 5X-aminodextran and mixed, preferably by means of a roller mixer, for about three (3) hours. After the mixing was completed, the pH was adjusted to about 10.0 by the addition 2 $\mu$L of 5M aqueous potassium hydroxide. Immediately after the pH adjustment, 0.117 ml of 25% glutaraldehyde solution (0.311 moles or 1 mole per mole amino groups in 5X-aminodextran) was added to the bead containing solution and mixed therewith, preferably by roller mixing, for about one hour. The yellow color indicative of Schiff's base formation was noted after 15 minutes of mixing. The beads were then separated from free glutaraldehyde and aminodextran by centrifugation, the supernatant liquid discarded. To block unreacted aldehyde groups, the beads were resuspended in 10 ml of 1% w/v 5X-aminodextran and mixed, preferably by roller mixing, for a time in the range of about 8-16 hours. Alternatively, ethylenediamine (10 moles per mole gluteraldehyde) can be used as the blocking reagent. The Schiff's base formed by these reactions was reduced by the addition of 2.35 ml of 10 mg/ml sodium borohydride to the bead mixture and mixing for about an additional thirty minutes. Subsequently, the beads were washed four times using centrifuging and 1X PBS. The wash supernatant liquids were discarded and the washed beads were suspended in sufficient 1X PBS to give a 10 ml total volume suspension of either 5X-aminodextran coated carboxylated beads or 5X-aminodextran coated aldehyde/sulfate beads. Beads coated with 1X-, 2X- and 3X-aminodextrans may be similarly produced. Crosslinking 3X-Aminodextran on Sulfated Polystyrene Latex Particles.

Polystyrene latex particles which lack chemically reactive functional groups with which an aminodextran can bind quire the use of a modified coating and crosslinking procedure. The sulfated polystyrene latex particles used in this procedure do not have the aldehyde groups which are present on aldehyde/sulfate particles.

16.7 ml of 30 mg/ml aqueous 3X-aminodextran and 77.2 ml of distilled water were added to 6.1 ml of an 8.2% w/v solid sulfated polystyrene latex particle suspension. (2.13 $\mu$m mean particle diameter IDC) in a 250 ml tissue culture flask. The pH of the resulting suspension was then adjusted to 10.0 by the dropwise addition of 0.1M aqueous sodium hydroxide (about 20 drops were used). The flask was placed on an orbital shaker and its contents mixed for about 8-16 hours. After mixing, 1.130 ml of 25% glutaraldehyde solution (3 mg glutaraldehyde per milliliter reaction volume) were added to the bead suspension which was then orbitally shaker for an additional 30 minutes. Unreacted aldehyde groups were then blocked by the addition of 2 ml of ethylenediamine (10 mole per mole glutaraldehyde), followed by further shaking for about three hours. Finally, 1 ml of aqueous 10 mg/ml sodium borohydride and 1 mM potassium hydroxide solution was added to the latex particle suspension which was then orbitally shaken for about an additional thirty minutes in order to reduce and stabilize the Schiff's base linkages. The latex particles were then separated and washed four times using centrifugation and 1X PBS respectively. The beads were resuspended in 1X PBS and the volume adjusted to make a 2.5% w/v solids suspension (final volume about 20 ml) which can be used in subsequent reactions.

Particle Activation by Sulfo-SMCC and Antibody Coupling to the 5X-Aminodextran Coated Polystyrene.

The conjunction of monoclonal antibodies to the aminodextran coated polystyrene latex particles was performed as described herein for gelatin coated latex particles except that 33.75 µL of 10 mg/ml Sulfo-SMCC solution was used per milliter of 2.5% w/v solids bead suspension. The antibody conjugated, aminodextran coated polystyrene latex particles prepared according to the invention may be used in various cell population analyses by flow cytometry and other methodologies. For examples, see International patent application Publication WO 90/10313.

Unless specified otherwise, the quantity of monoclonal antibody conjugated to beads is in the range of 3-5 mg of antibody per square meter bead surface area. The beads are prepared, for example, by the reaction of an activated antibody such as an iminothiolane activated antibody with activated beads. Typically, the reaction uses 0.625 mg of activated antibody per milliliter of 1% w/v bead suspension.

Example 4. Polystyrene Beat T-cell Population Shift Analysis by Flow Cytometry Using Aminodextran Coated Polystyrene Latex Beads.

A 50 ml sample of $Na_4$ EDTA-anticoagulated blood was obtained, divided among a plurality of centrifuge tubes and centrifuged at 500g for ten (10) minutes. The majority of the plasma was removed and the residual buff colored layer of cells in each tube was pooled and further centrifuged at 500g for an additional 10 minutes. The residual plasma and the buff colored cells were separated. Collectively, the buff colored cells and the plasma constitute the leuko-rich whole blood.

The buff colored cells were diluted with 1X PBS to obtain a plurality samples containing 4, 6, 8, 10 and 12 x $10^5$ white blood cells (wbc), respectively, as determined on a Coulter S-Plus® counter. 100 µL aliquots of each of the 4, 6, 8, 10 and 12 x $10^5$ wbc samples were then pipetted into a plurality of 5 ml test tubes. Subsequently, 5, 10, 15, 20, 30 and 40 µL aliquots of a suspension of T4 monoclonal antibody conjugated to 3X-aminodextran coated sulfate polystyrene latex beads (1% w/v solids, 3-5 mg antibody per square meter bead surface area and 1.786 X $10^9$ particles per milliliter) was then pipetted into a separate tube for each of the wbc concentrations. Each tube was then nutated for two minutes. Following nutation, 10 µL of CYTO-STAT® T4-RD1/T8-FITC dual color antibody reagent was added to each sample to enable discrimination between actual t4+ cell labelling and non-specific labelling of T-cells, i.e., T8 cells. The samples were vortexed and incubated at room temperature (range 18-27°C) for ten minutes. The sample tubes were then placed on a Coulter EPICS Q-Prep® (Coulter Corporation, Miami, Florida) or similar instrument and processed on the thirty-five second lyse mode to lyse red blood cells (rbc). After rbcs were lysed and the samples fixed (Q-Prep), all samples were analyzed on a Coulter EPICS® Profile II flow cytometer or similar instrument. Standard programs introduced into the Profile were used to determine lymphocyte and moncyte-myeloid population as fluorescent events; in particular, those lymphocytes which are in the shifted-forward versus side-scatter region due to a change in size and/or granularity upon binding of the polystyrene latex particles.

The Profile II was calibrated with DNA-Check (Coulter Corporation, Miami, Florida) beads for alignment and with Standard-Brite beads, (Coulter Corporation) for fluorescence intensity. A buff cell sample containing no polystyrene beads and no fluorescent markers was used to establish the population (quantity) of lymphocytes, monocytes and granulocytes. The lymphocyte population was Bitmapped for fluorescence analysis and all three cell type populations were boxed for cell count as fluorescent events. A buff cell sample without polystyrene beads was stained with CY-TO-STAT® MsIgG1-RD1/Ms IgG1-FITC control reagent and used to set quad stats and cursors. A buff cell sample containing the dual fluorescent reagent T4-RD1/T8-FITC and no polystyrene beads was used to set color compensation. A buff cell sample containing 40 µL of T4-conjugated polystyrene latex particles was used to determine the position of the shifted cell population which was Bitmapped and boxed. A buff cell sample of T4-conjugated polystyrene latex particles was stained with MsIgG1-RD1/MsIgG1-FITC control reagent and used to set quads and cursors. A sample of T4-conjugated polystyrene latex beads with no buff cells or fluorescent reagents present was used to verify that the beads did not interfere with cells on the scatter-gram. Upon completion of the above steps, buff cell samples which contain the various wbc concentrations and the dual color markers (RD1 and FITC) were used to determine an original T4 cells fluorescent event count. Lastly, a buff cell sample with dual color mark and various amounts (5, 10, 15, 20, 30 and 40 µL) of the T4 containing beads of concentration 3-5 mg T4/m$^2$ bead surface area were used to determine the T4 cell fluorescent event cell count in the region of the forward-scatter versus side-scatter plot shifted away from the remaining lymphocyte population in the direction of greater size and/or granularity.

In the preparation of the test and control samples, it is important that antibody-bead conjugates be mixed with the buff cell sample before the addition of the fluorescent markers in order to avoid saturation of antigenic sites on the T cells by the smaller and more mobile molecular dye-antibody conjugates. The T4 cell counts were estimated from the T4 fluorescent events and from S-Plus® counter versus Profile II data, [T4 events (no beads) - T4 events (40 µL beads)] x [Q-Prep Dilution Factor(11)] x [(S-Plus lymphocyte count) ÷ (total lymphocyte events)=1.24.] The results are summarized in Table 10 below and in FIG. 1 as the number of T4 cells shifted versus the bead-to-T4 cell ratio.

Table 10.

| WBC/100 µL (x 10$^5$) | T4 cells Shifted (x10$^4$) | %T4 in WBC | %T4in Lymphocytes | Bead/T4 cell (at Fig. 1 Plateau) |
|---|---|---|---|---|
| 12.0 | 18.4 | 15.3 | 39.3 | 147 |
| 10.0 | 15.3 | 15.3 | 39.3 | 234 |
| 8.0 | 11.9 | 14.9 | 38.3 | 302 |
| 6.0 | 8.80 | 14.7 | 37.8 | 603 |
| 4.0 | 6.00 | 15.0 | 38.6 | 884 |

The percentage of lymphocytes in the wbc sample was 38.9% as determined by the S-Plus® counter. The percentage of T4 cells among the lymphocytes was determined by the equation:

$$\%T4 \text{ cells} = 100\% \times [(\text{number of T4 cells in shifted}$$

$$\text{population at plateau in plot}) \div \text{wbc count}] \div 0.389].$$

The T4 percentages were 39.3%, 39.3%, 38.3%, 37.8% and 38.6% for wbc concentrations of 12, 10, 8, 6 and 4 x 10$^5$, respectively. The T4 percentages based on relative fluorescence events of shifted and unshifted lymphocyte population, [(T4 events (no beads) - T4 events (40 µl BEADS)] ÷ (total lymphocyte events), in forward-scatter versus side-scatter plot were 39.2, 39.5, 38.4, 38.0 and 39.0, respectively. Similar experiments with T4 monoclonal antibody conjugated to sulfate polystyrene latex beads coated with type A gelatin showed interference from shifted monocytes due to non-specific interactions with crosslinked gelatin. The use of minodextran coated polystyrene latex beads thus provides a viable alternative when such interference is observed.

Example 5. T4 and T8 Cell Population Analysis Using 5X-Aminodextran Coated Polystyrene Latex Particles.

T4 and T8 monoclonal antibodies were conjugated to 5X-aminodextran coated carboxylate polystyrene latex particles and 5X-aminodextran coated aldehyde/sulfate polystyrene latex particles. The particles were suspended in 1X PBS to give a 1% w/v mixture. A sufficiency of T4 or T8 conjugated particles was added to 200 µL whole blood. The sample volume was made up to 300 µL by the addition of 1X PBS and then vortexed for two minutes. A 100 µL aliquot was then aspirated into a Coulter VCS® (Coulter Corporation, Miami, Florida) or similar instrument for analysis of the population of shifted T-cells using the methods described by T. Russell et al. in International Patent Application Publication WO90/18013. The addition of batch lyse for red blood cells and of lyse quencher was automatically controlled by the instrument. The percent of T4 or T8 cells in the sample equals the T cell population shifted into an uncluttered region of DC versus opacity. The %T4 of T8 cells = [(shifted T4 or T8 cell population) ÷ (total original lymphocyte population)] x 100%. The plot from which you obtain this percentage is DC versus [(RF-85) ÷ DC], where RF equals radio frequency scatter, DC equal direct current cell resistance and -85 is a constant. The quantity of monoclonal antibody conjugated, coated particles (1% w/v solids) used and the percentage T4 cells determined are shown in Table 11.

Table 11.

| A. Using Carboxylated Polystyrene Latex Particles. | |
|---|---|
| Bead Volume Used (µL) | % T4 cells |
| 0 | 0.00 |
| 5 | 18.94 |
| 10 | 27.61 |
| 20 | 36.76 |
| 30 | 39.30 |
| 40 | 41.17 |
| 50 | 40.84 |
| 75 | 44.07 |
| 100 | 44.13 |
| B. Using Aldehyde/Sulfate Polystyrene Latex Beads. | |
| Volume Beads Used (µL) | % T4 cells |
| 5 | 28.91 |
| 10 | 29.89 |
| 20 | 34.80 |
| 30 | 38.57 |
| 40 | 39.99 |
| 50 | 40.33 |
| 75 | 42.62 |
| 100 | 44.37 |

Using either type of particles, a plateau at about 44.1-44.4% T4 cells was observed when all T4 cells were shifted by an excess of polystyrene beads coated with T4 antibody. This end point value can be considered the true percentage of T4 cells among the lymphocyte population of the whole blood sample analyzed. The bead-to-T4 cell ratios at the end points were 1210 and 928, respectively.

Coupling of 1X-Aainodextran to Tosyl-Activated Polystyrene Magnetite-Containing Particles (No Crosslinking).

2 ml of 30 mg/ml uniform, porous polystyrene particles having embedded magnetic material (M-450 Dynabeads®, tosyl activated. 4.5 µm ± 5%; Dynal, Incorporated) were washed three times with 0.25M borate buffer, pH 9.8. 0.198 ml of 45.4 mg/ml 1X-aminodextran was added to the washed beads and the total volume was adjusted to 2.4ml with the borate buffer to give a 2.5% w/v solids suspension having an 1X-aminodextran concentration of 3.75 mg/ml. The suspension was then mixed for about 8-16 hours. The particles were then magnetically separated, washed four times with 2.4 ml of 1X PBS and resuspended in 1X PBS to give a particle suspension of total volume 2.4 ml.

T11 monoclonal antibody was conjugated to the 1X aminodextran coated M-450 beads using the method described herein for conjugating monoclonal antibodies to gelatin coated ferrite particles. The antibody conjugated particles were then used in a T11/B4 lymphoid cell assay performed using the method of Reference Example 1. The results are summarized in Table 12 for samples treated with 0, 25, 50 and 100 µL of T11 antibody-conjugated M-450 particles. The results indicate there was no significant non-specific removal of B cells. The bead-to-cell ratio was 48: 1 when 100 µL of particles were used.

Table 12.

| µL T11 Beads Used | T11+Count | B4+ Count |
|---|---|---|
| 0 (Control) | 18,470 | 2,643 |
| 25 | 1,680 | 2,709 |
| 50 | 1,348 | 2,999 |
| 100 | 291 | 2,675 |

Coupling 5X-Aminodextran to Polystyrene-Magnetite Particles.

50 ml of polystyrene-magnetite particles (10% w/v solids in 0.5% SDS, 0.980 μm means diameter, -COOH functional group, 23.1% magnetite) were added to a reaction flask containing 150 ml of 1% sodium azide, 10 mM sodium bicarbonate solution. The contents were mixed for about 8-16 hours using shaker and the particles then twice magnetically separated and washed using 200 mL of the 10 mM aqueous sodium bicarbonate. The washed particles were then suspended in 0.2M aqueous sodium chloride and the suspension volume adjusted to about 150 ml. A solution of 750 mg 5X-aminodextran in about 10 ml of 0.2M aqueous sodium chloride was prepared and added to the particle suspension. The total volume was then adjusted to 200 ml with 0.2M aqueous sodium chloride to give a 2.5% w/v solids suspension having a 5X-aminodextran concentration of 3.75 mg/ml. A 1.80 ml sample of 10 mg/ml EDAC in 0.2M aqueous sodium chloride was then added to the particle suspension to facilitate coupling aminodextran amine groups with the -COOH group present on the particle's surface. The EDAC containing suspension was mixed on the orbital shaker for about 8-16 hours and then magnetically separated and washed three times with distilled water. The washed 5X-aminodextran coated beads were then resuspended in sufficient 1X PBS to yield 200 mL of 2.5% w/v solids suspension.

Activation of 5X-Aminodextran-Coated Polystyrene-Magnetic Particles by Sulfo-SMCC.

Except for the following differences, monoclonal antibodies were conjugated to the 5X-aminodextran-coated polystyrene-magnetite particles by the procedures described herein for conjugating monoclonal antibodies to gelatin coated particle.

1. Activation of the 5X-aminodextran coated particles was done using 33.75 μL of 10 mg/ml sulfo-SMCC solution per milliliter of 2.5% w/v solids suspension.
2. Antibody-particle coupling was done using about 0.313 mg iminothiolane-activated monoclonal antibody per milliter of 1.25% w/v solids suspension of activated particles.

The activation of beads by iminothiolane and antibodies by sulfo-SMCC may be reversed or other reagents used as activating as specified elsewhere herein.

Example 6. Test Results Summary for Antibodies Conjugated to 5X-Aminodextran Coated Particles.

The following assay results were obtained using the protocols described in Reference Examples 1-3.

A. Erythrocyte/Thrombocyte Assay.

An erythrocyte/thrombocyte assay was performed using 0-250 μL of 0.8333% w/v "solids. KC-16 conjugated magnetic beads. The results are summarized in Table 13. The results indicate that 100 μL pf 0.833% w/v" beads contains $1.32 \times 10^9$ particles which remove $4.9 \times 10^7$ rbcs from assay sample, thus yielding a bead-to-cell ratio of 27.

Table 13.

| RBC Depletion Using Magnetic Beads | | | |
|---|---|---|---|
| μL Beads Used | wbc x $10^4$ | rbc x $10^7$ | platelets x $10^6$ |
| 0 | 7.0 | 4.9 | 2.5 |
| 5 | 5.0 | 4.3 | 2.1 |
| 10 | 6.0 | 3.9 | 1.8 |
| 20 | 6.0 | 3.0 | 1.6 |
| 40 | 6.0 | 1.1 | 1.6 |
| 60 | 6.0 | 0.5 | 2.2 |
| 80 | 6.0 | 0.2 | 2.2 |
| 100 | 6.0 | <0.1 | 2.2 |
| 150 | 6.0 | <0.1 | 2.0 |
| 200 | 6.0 | <0.1 | 2.1 |
| 250 | 5.0 | <0.1 | 2.2 |

B. T4/T8 Lymphoid Cell Assay.

A T4/T8 lymphoid depletion assay was carried out using 0-250 μL of 0.025% w/v solids, T4 monoclonal antibody conjugated magnetic beads. The results are summarized in Table 14. After scaling the total lymphocyte fluorescent events to the number of lymphocytes measured on a Coulter S-Plus® Counter, the number of T4 cells remaining after each depletion was estimated and these data were used to show that 150 μL of 0.25% w/v solids beads contains $6.0 \times 10^7$ particles which removed $6.0 \times 10^5$ T4 cells, thereby yielding a bead-to-cell ratio of 100.

Table 14.

| T4 Cell Depletion Using Magnetic Beads | | |
|---|---|---|
| μL Beads Used | T4 | T8 |
| 0 | 40,920 | 26,278 |
| 5 | 30,058 | 24,169 |
| 10 | 25,920 | 23,097 |
| 30 | 21,238 | 22,467 |
| 40 | 8,630 | 23,183 |
| 60 | 2,990 | 22,384 |
| 80 | 1,985 | 22,482 |
| 100 | 1,096 | 22,482 |
| 150 | 108 | 22,680 |
| 200 | 40 | 22,816 |
| 250 | 16 | 22,627 |

C. Erythrocyte/Thrombocyte Assay

An erythrocyte/thrombocyte assay was performed using 0-250 μL of 0.25% w/v solids, PLT-1 monoclonal antibody conjugated magnetic beads. The results are summarized in Table 15. The results indicate that 200 μL of 0.25% w/v solids beads contains $7.92 \times 10^8$ particles which removed $9.2 \times 10^6$ platelets, thereby yielding a bead-to-platelet ratio of 8.6.

Table 15.

| Platelet Depletion Using Magnetic Beads | | | |
|---|---|---|---|
| μL Beads Used | wbc x $10^5$ | rbc x $10^7$ | platelets x $10^6$ |
| 0 | 2.3 | 5.3 | 9.2 |
| 5 | 2.3 | 5.3 | 7.7 |
| 10 | 2.3 | 5.3 | 7.0 |
| 20 | 2.3 | 5.3 | 5.5 |
| 40 | 2.2 | 5.3 | 3.4 |
| 60 | 2.3 | 5.3 | 1.7 |
| 80 | 2.2 | 5.3 | 1.0 |
| 100 | 2.2 | 5.3 | 0.4 |
| 150 | 2.2 | 5.3 | 0.1 |
| 200 | 2.2 | 5.4 | <0.1 |
| 250 | 2.2 | 5.1 | <0.1 |

D. Myeloid Cell Assay.

A myeloid cell assay was performed using 0-250 μL of 0.125% w/v, MO2 monoclonal antibody conjugated magnetic beads. The results are summarized in Table 16. The results indicate that 200 μL of 0.125% w/v solids beads contains $4.0 \times 10^8$ particles which were estimated to remove $4.8 \times 10^4$ monocytes, thereby yielding a bead-to-monocyte ratio of $8.3 \times 10^3$.

Table 16.

| Monocyte Cell Depletion Using Magnetic Beads | | | |
|---|---|---|---|
| | Counts | | |
| µL Beads Used | Lymphocytes | Monocytes | Granulocytes |
| 0 | 78,931 | 5,958 | 118,649 |
| 5 | 78,318 | 4,297 | 117,991 |
| 10 | 78,836 | 4,128 | 118,943 |
| 20 | 76,447 | 3,978 | 118,642 |
| 40 | 76,386 | 2,308 | 121,560 |
| 60 | 74,597 | 1,700 | 121,831 |
| 80 | 75,069 | 1,013 | 118,895 |
| 100 | 76,837 | 800 | 117,794 |
| 150 | 76,613 | 368 | 116,520 |
| 200 | 78,018 | 214 | 118,762 |
| 250 | 78,817 | 228 | 119,026 |

E. Myeloid Cell Assay.

A myeloid cell assay was performed using 0-250 µL of 0.1% w/v solids, KC-48 monoclonal antibody conjugated beads. The results are summarized in Table 17. The results indicate that 150 µL of 0.1% w/v solids beads contains $2.4 \times 10^8$ particles which were estimated to remove $1.6 \times 10^6$ granulocytes, thereby yielding a bead-to-granulocyte ratio of 149.

Table 17.

| Granocyte Cell Depletion Using Magnetic Beads | | | |
|---|---|---|---|
| | Counts | | |
| µL Beads Used | Lymphocytes | Monocytes | Granulocytes |
| 0 | 92,098 | 11,028 | 81,738 |
| 10 | 91,543 | 7,001 | 9,653 |
| 50 | 88,791 | 5,856 | 2,547 |
| 100 | 88,669 | 5,717 | 1,849 |
| 150 | 88,924 | 5,948 | 1,409 |
| 200 | 89,738 | 5,697 | 1,316 |
| 250 | 81,183 | 5,029 | 1,316 |

A similar depletion study was conducted using the same control samples as Table 17 and 0-250 µL of 0.5% w/v solids, 1D3 monoclonal antibody conjugated magnetic beads. These results are summarized in Table 18. The results indicate that 50 µL of 0.5% w/v solids beads contains $4.0 \times 10^8$ particles which were estimated to remove $1.54 \times 10^6$ neutrophils, thereby giving a bead-to-neutrophil ratio of 260.

Table 18.

| Neutrophil Cell Depletion Using Magnetic Beads | | | |
|---|---|---|---|
| | Counts | | |
| µL Beads Used | Lymphocytes | Monocytes | Granulocytes |
| 0 | 92,098 | 11,028 | 81,738 |
| 10 | 87,708 | 8,617 | 7,084 |
| 50 | 87,288 | 7,306 | 4,688 |
| 100 | 89,300 | 7,717 | 4,952 |
| 150 | 80,257 | 7,468 | 3,896 |

Table 18.   (continued)

| Neutrophil Cell Depletion Using Magnetic Beads | | | |
|---|---|---|---|
| | Counts | | |
| μL Beads Used | Lymphocytes | Monocytes | Granulocytes |
| 200 | 79,666 | 6,185 | 4,691 |
| 250 | 75,726 | 5,070 | 4,529 |

F. T11/B4 Lymphoid Cell Assay.

A T11/B4 lymphoid cell assay was performed using 0-60 μL of 0.25% w/v solids, T11 monoclonal antibody conjugated magnetic beads. The results are summarized in Table 19. The results indicate that 20 μL of 0.25% w/v solids beads contains $8.0 \times 10^7$ particles which were estimated to remove $6.46 \times 10^5$ T11+ cells, thereby yielding a bead-to-T11+ cell ratio of 124.

Table 19.

| T11+ Cell Depletion Using Magnetic Beads | | | | |
|---|---|---|---|---|
| | Counts | | | |
| μL Beads Used | T11+ | B4+ | Monocytes | Granulocytes |
| 0 | 40,112 | 7,728 | 14,569 | 62,540 |
| 5 | 17,919 | 8,352 | 10,227 | 82,620 |
| 10 | 8,789 | 8,160 | 5,974 | 62,268 |
| 20 | 791 | 7,801 | 5,568 | 69,571 |
| 40 | 622 | 7,846 | 4,088 | 63,587 |
| 60 | 429 | 7,550 | 2,812 | 57,175 |

A second depletion study was performed using the same control sample as used in Table 19 and samples depleted using 5-60 μL of 0.25% w/v solids, B4 monoclonal antibody conjugated magnetic beads. The results are summarized in Table 20. The results indicate that 40 μL of 0.25% w/v solids beads contain $1.6 \times 10^8$ particles which were estimated to remove $1.23 \times 10^5$ B4+ cells, thereby, yielding a bead-to-B4+ cell ratio of 1300.

Table 20.

| B4+ Cell Depletion Using Magnetic Beads | | | | |
|---|---|---|---|---|
| | Counts | | | |
| μL Beads Used | T11+ | B4 | Monocytes | Granulocytes |
| 0 | 40,112 | 7,728 | 14,569 | 62,540 |
| 5 | 37,103 | 5,618 | 7,523 | 66,430 |
| 10 | 33,754 | 2,587 | 8,158 | 65,198 |
| 20 | 33,393 | 888 | 7,656 | 59,380 |
| 40 | 33,473 | 131 | 8,846 | 64,326 |
| 60 | 32,956 | 43 | 5,557 | 52,345 |

G. Myeloid Cell Assay Using Magnetic Beads.

A myeloid cell depletion assay of wbcs was performed using 0-250 μL of 0.5% w/v solids, KC-56 monoclonal antibody conjugated magnetic beads. The results are summarized in Table 21. The results indicate that 250 μL of beads contains $1.98 \times 10^9$ particles which were estimated to remove $1.35 \times 10^6$ wbcs, thereby yielding a bead-to-wbc ratio of 1470.

Table 21

| WBC Depletion | | | |
|---|---|---|---|
| | Counts | | |
| µL Beads Used | Lymphocytes | Monocytes | Granulocytes |
| 0 | 50,616 | 10,238 | 24,526 |
| 5 | 26,574 | 4,962 | 36,066 |
| 10 | 12,922 | 2,821 | 32,079 |
| 20 | 4,967 | 2,031 | 30,468 |
| 40 | 1,724 | 1,017 | 22,529 |
| 60 | 671 | 630 | 16,670 |
| 80 | 473 | 450 | 13,167 |
| 100 | 343 | 304 | 11,456 |
| 150 | 144 | 185 | 7,446 |
| 200 | 75 | 102 | 5,474 |
| 250 | 5 | 1 | 65 |

**Claims**

1. Discrete colloidal particles having a surface coating having a plurality of pendant functional groups, the particles comprising:

    (a) a solid, non-gelatin, non-aminodextran core
    (b) a first coating of at least one substance selected from

        (i) a type B, alkali cured gelatin of Bloom in the range 60 to 225 and
        (ii) a type A, acid cured gelatin of Bloom in the range 60 to 300, and
        (iii) an aminodextran;

    (c) when the coating is type A or type B gelatin, a second, aminodextran coating; and
    (d) pendant functional groups covalently attached to the outermost coating;

     wherein the or each coating on the core is either:

    (I) crosslinked by the action of a chemical crosslinking agent, or
    (II) not crosslinked or crosslinked by a chemical crosslinking agent when the outermost coating is an aminodextran that is covalently coupled to the core.

2. Particles according to claim 1, wherein the core is selected from

    (i) only a magnetic substance,
    (ii) a polymeric substance, either magnetic or non-magnetic, which has surface amine-reactive groups, and
    (iii) a polymeric substance, either magnetic or non-magnetic, which does not have surface amine-reactive functional groups;

     and wherein the or each coating on the core is either:

    (I) crosslinked by the action of a chemical crosslinking agent when the core does not have surface amine-reactive groups, or
    (II) not crosslinked or crosslinked by a chemical crosslinking agent when the first coating is an aminodextran and the core has surface amine-reactive groups, the aminodextran being covalently coupled to the core surface by reaction between the amine-reactive groups and amine groups on the aminodextran.

3. Particles according to claim 1 or claim 2, wherein the core is a polymeric substance and only the first coating is

**34**

present and is an aminodextran.

4. Particles according to claim 2, wherein the core is selected from a polymer only substance or a polymeric magnetic substance and the first coating is an aminodextran.

5. Particles according to claim 1 or claim 2, wherein the first coating comprises a type B gelatin and the second coating comprises an aminodextran, either both coating layers being crosslinked on the core surface or the first coating being adsorbed onto the substrate with the second coating joined thereto by a condensation reaction.

6. Particles according to any preceding claim, wherein the core comprises a magnetic substance.

7. Particles according to claim 6, wherein the surface of the core is hydrophobic.

8. Particles according to any preceding claim, wherein the core is in the size range of 0.1 to 5.0 microns.

9. Particles according to claim 8, wherein the core is in the size range of 0.1 to 1.0 microns.

10. Particles according to any preceding claim, wherein the core comprises polystyrene latex.

11. Particles according to any preceding claim, wherein the core surface has amine-reactive functional groups selected from the group consisting of aldehyde, carboxylic acids and esters, and tosyl groups.

12. Particles according to any preceding claim wherein the crosslinking agent is glutaraldehyde.

13. Particles according to any preceding claim, wherein the aminodextran is selected from 1X-, 2X-, 3X- and 5X-aminodextran.

14. Particles according to claim 13, wherein the aminodextran is 5X-aminodextran.

15. Particles according to any preceding claim, wherein the functional groups pendant from the outermost coating are selected from amino groups, maleimidyl groups, sulfhydryl groups and a biological substance.

16. Particles according to claim 15, wherein the pendant functional groups are biological substances selected from monoclonal antibodies, polyclonal antibodies and biological cells.

17. Particles according to claim 16, wherein the functional groups are polyclonal antibodies.

18. Particle according to claim 16, wherein the functional groups are monoclonal antibodies.

19. Particles according to claim 15, wherein the pendant functional groups are amino groups.

20. Particles according to claim 15, wherein the pendant functional groups are selected from maleimidyl groups and sulfhydryl groups.

21. Particles according to claim 20, wherein a biological substance is bound to either the maleimidyl groups or the sulfhydryl groups.

22. Particles according to claim 21, wherein the biological substance is as defined in any of claims 16 to 18.

23. Particles according to any of claim 21 or claim 22, wherein the biological substance comprises reactive substituents selected from sulfhydryl substituents and maleimidyl susbtituents, provided that when the particle 5 pendant functional groups are maleimidyl groups the reactive substituents are sulfhydryl groups, and when the particle pendant functional groups are sulfhydryl groups the reactive substituents are maleimidyl groups.

24. Particles according to claim 15, wherein the functional groups comprise a biological substance which is bonded to the outermost coating of the core.

25. Particles according to claim 24, wherein the biological substance is bonded to the outermost coating through a bifunctional bridging group.

26. Particles according to claim 25, wherein the bridging group comprises a functional group as defined in claim 16 or claim 17, preferably being a polyamine having an amine group bonded to the outermost coating and another amine group or groups bonded to a moiety having a reactive maleimidyl or sulfhydryl group, the polyamine being selected from ethylenediamine, 1,3-diaminopropane, 1,4-cyclohexanediamine, 1,4-cyclohexenediamine, 1,4-phenylenediamine, diethylene triamine and aminodextrans.

27. Particles according to any of claims 23 to 26, wherein the sulfhydryl groups are naturally present on the biological substance or are generated by modification of an amino group or groups naturally present on the biological substance using 2-iminothiolane hydrochloride, and the maleimidyl groups are generated by modification of an amino group or groups on the biological substance using a maleimidyl-containing reagent.

28. A process for preparing colloidal particles as defined in claim 1, the process comprising:

    (a) coating the core with

        (i) a type B alkali cured gelatin of Bloom in the range 60 to 225;
        (ii) a type A, acid cured gelatin of Bloom in the range 60 to 300; or
        (iii) an aminodextran;

    (b) coating the product of steps (a) (i) or (a)(ii) with an aminodextran;
    (c) either (i) not crosslinking the coating of the product of step (a)(iii) when the aminodextran is covalently coupled to the core, or (ii) crosslinking the or each coating of the products of steps (a)(iii) or (b) with a chemical crosslinking agent;
    (d) blocking any free, unreacted crosslinking agent functional groups present by reacting said groups with a polyamine, at least one polyamine amino group remaining unreacted; and
    (e) washing the product of step (d) or step (c) (i).

29. A process according to claim 28, wherein the product of step (e) is reacted with at least one substance selected from:

    (i) a reagent which will result in the product having pendant sulfhydryl groups,
    (ii) a reagent which will result in the product having pendant maleimidyl groups, and
    (iii) a reagent which will result in the product having pendant biological substances, preferably as defined in any of claims 16 to 18 or claim 23.

30. A process according to claim 28 or claim 29, wherein the polyamine is as defined in claim 26.

31. A process according to any of claims 28 to 30, wherein in step (a) when the core substance is a magnetic substance it is coated with a type B gelatin; when the core substance is a polymeric substance not having surface amine-reactive functional groups it is coated with a type A gelatin; and when the core substance is a polymeric substance having amine-reactive functional groups it is coated with an aminodextran, and in step (c) (i) the aminodextran is covalently coupled to the amine-reactive groups on the core surface.

32. A process according to any of claims 28 to 31, wherein the core is metallic, the process comprising in step (a):

    (i) preparing metallic core particles in a gelatin, preferably a type B gelatin; or
    (ii) adsorbing, as a first coating, a gelatin onto pre-formed metallic particles and adsorbing, as a second coating, an aminodextran onto the surface of the gelatin-coated particles; or
    (iii) adsorbing, as a first coating, a gelatin onto pre-formed metallic particles and joining to the gelatin, by a condensation reaction, an aminodextran as a second coating,

    the process further optionally comprising, after washing, derivatising the resultant particles by reaction with a bifunctional bridging reagent to incorporate pendant functional groups, the functional groups preferably being as defined in claim 29.

33. A process according to claim 32, wherein the resultant particles are reacted with a bifunctional bridging agent.

**34.** A process according to claim 33, wherein the gelatin is a type B gelatin and wherein (iii) of claim 32 is carried out, by mixing core material with a 1% w/v aqueous solution of gelatin, and wherein the reaction with the bifunctional bridging reagent is carried out in phosphate buffered saline solution at ambient temperature for 0.5 to 1.5 hours to prepare particles having reactive terminal maleimidyl or sulfhydryl groups bound to the particles' surface, the process further comprising the following steps:

(i) preparing an antibody for bonding to the particles by generating reactive substituents on that antibody which are selected from sulfhydryl groups and maleimidyl groups;

(ii) reacting the particles and the antibody, with mixing, for 1 to 3 hours, thereby coupling the reactive substituents on the antibody to the functional groups on the particles, and separating the resulting antibody-containing particles from the reaction medium and washing with buffered saline solution;

(iii) blocking unreacted groups present on the product of step (ii) above; and

(iv) separating and washing the antibody-containing particles with about 1% bovine serum albumin in 0.1% $NaN_3$ in phosphate buffered saline solution, storing the washed particles in that solution at about 4°C for 8 to 16 hours, re-washing the particles and storing until required for use.

**35.** A process according to claim 34, wherein (ii) of claim 32 is carried out, by washing the gelatin-coated particles with a solution of an aminodextran, storing the particles for up to about six months before suspending the particles in an aminodextran coating solution, the process further comprising, prior to reaction of the particles with the bifunctional bridging reagent, the following steps:

(i) mixing the particle suspension with a solution of glutaraldehyde for about 1 hour, thereby crosslinking the gelatin/aminodextran surface;

(ii) adding ethylenediamine to the suspension and mixing for 1 to 4 hours;

(iii) adding $NaBH_4$ to the suspension and mixing;

(iv) separating the particles from the suspension and washing with 0.2 M aqueous NaCl;

(v) reacting, with mixing, the particles of step (iii) or (iv) above with ethylenediamine in 0.2 M NaCl aqueous solution containing 1-ethyl-3-(3-diethylaminopropyl) carbodiimide at ambient temperature; and

(vi) separating the particles and washing them with phosphate buffered saline solution.

**36.** A process according to any of claims 28 to 31, wherein the core is a polymeric substance having or not having amine-reactive functional groups, the process further comprising:

(A) coating a gelatin, preferably a type A gelatin, or an aminodextran onto the core surface either by:

(i) adsorbing the gelatin or aminodextran onto the core surface or

(ii) covalently coupling the aminodextran to the core by reacting the aminodextran amine groups with the amine-reactive groups on the core;

B) (i) crosslinking the gelatin or aminodextran coating of step (A)(i) using a chemical crosslinking agent, or (ii) not crosslinking or selectively crosslinking with a chemical crosslinking agent the aminodextran coating of step (A)(ii), where present.

**Patentansprüche**

**1.** Diskrete kolloidale Teilchen mit einer Oberflächenbeschichtung, welche eine Vielzahl seitenständiger funktioneller Gruppen aufweist, wobei die Teilchen umfassen:

(a) einen festen Nicht-Gelatine, Nicht-Aminodextran-Kern;

(b) eine erste Schicht aus mindestens einer Substanz, ausgewählt aus

(i) einer alkaligehärteten Gelatine vom Typ B mit einer Gelstärke nach Bloom von 60 bis 225 und

(ii) einer säuregehärteten Gelatine vom Typ A mit einer Gelstärke nach Bloom im Bereich von 60 bis 300 und

(iii) einem Aminodextran;

(c) wenn die Beschichtung eine Gelatine vom Typ A oder B ist, eine zweite Aminodextranschicht und

(d) seitenständige funktionelle Gruppen, kovalent an die äußerste Schicht gebunden, wobei die oder jede Beschichtung auf dem Kern entweder:

(I) mittels eines chemischen Vernetzungsmittels vernetzt ist oder
(II) nicht vernetzt ist oder mittels eines chemischen Vernetzungsmittels vernetzt ist, wenn die äußerste Beschichtung ein Aminodextran ist, welches kovalent an den Kern gebunden ist.

2. Teilchen nach Anspruch 1, wobei der Kern aus

(i) nur einer magnetischen Substanz,
(ii) einer Polymersubstanz, entweder magnetisch oder nicht magnetisch, die aminreaktive Oberflächengruppen aufweist, und
(iii) einer Polymersubstanz, entweder magnetisch oder nicht magnetisch, welche keine aminreaktiven Oberflächengruppen aufweist, ausgewählt ist und wobei die oder jede Beschichtung des Kerns entweder:

(I) durch die Wirkung eines chemischen Vernetzungsmittels vernetzt ist, wenn der Kern keine aminreaktiven Oberflächengruppen aufweist, oder
(II) nicht vernetzt ist oder mittels eines chemischen Vernetzungsmittels vernetzt ist, wenn die erste Beschichtung ein Aminodextran ist und der Kern aminreaktive Oberflächengruppen aufweist, wobei das Aminodextran kovalent an die Kernoberfläche durch Reaktion zwischen den aminreaktiven Gruppen und den Amingruppen an dem Aminodextran gebunden ist.

3. Teilchen nach Anspruch 1 oder 2, wobei der Kern eine Polymersubstanz ist und nur die erste Beschichtung vorhanden ist und ein Aminodextran ist.

4. Teilchen nach Anspruch 2, wobei der Kern aus einer Substanz nur aus Polymer oder einer polymeren magnetischen Substanz ausgewählt ist und die erste Beschichtung ein Aminodextran ist.

5. Teilchen nach Anspruch 1 oder 2, wobei die erste Beschichtung eine Gelatine vom Typ B umfasst und die zweite Beschichtung ein Aminodextran umfasst, wobei entweder beide Beschichtungsschichten auf der Kernoberfläche vernetzt sind oder die erste Beschichtung auf dem Substrat adsorbiert ist und die zweite Beschichtung daran mittels einer Kondensationsreaktion gebunden ist.

6. Teilchen nach einem vorhergehenden Anspruch, wobei der Kern eine magnetische Substanz umfasst.

7. Teilchen nach Anspruch 6, wobei die Kernoberfläche hydrophob ist.

8. Teilchen nach einem vorhergehenden Anspruch, wobei der Kern eine Größe von 0,1 µm bis 5,0 µm aufweist.

9. Teilchen nach Anspruch 8, wobei der Kern zwischen 0,1 und 1,0 µm groß ist.

10. Teilchen nach einem vorhergehenden Anspruch, wobei der Kern Polystyrol-Latex umfasst.

11. Teilchen nach einem vorhergehenden Anspruch, wobei die Kernoberfläche aminreaktive funktionelle Gruppen, ausgewählt aus der Gruppe, bestehend aus Aldehyden, Carbonsäuren und Estern und Tosylgruppen, aufweist.

12. Teilchen nach einem vorhergehenden Anspruch, wobei das Vernetzungsmittel Glutaraldehyd ist.

13. Teilchen nach einem vorhergehenden Anspruch, wobei das Aminodextran aus 1X-, 2X-, 3X- oder 5X-Aminodextran ausgewählt ist.

14. Teilchen nach Anspruch 13, wobei das Aminodextran ein 5X-Aminodextran ist.

15. Teilchen nach einem vorhergehenden Anspruch, wobei die seitenständigen funktionellen Gruppen der äußersten Beschichtung aus der Gruppe der Aminogruppen, Maleimidylgruppen, Sulfhydrylgruppen und einer biologischen Substanz ausgewählt sind.

16. Teilchen nach Anspruch 15, wobei die seitenständigen funktionellen Gruppen biologische Substanzen, ausgewählt aus monoklonalen Antikörpern, polyklonalen Antikörpern und biologischen Zellen, sind.

17. Teilchen nach Anspruch 16, wobei die funktionellen Gruppen polyklonale Antikörper sind.

18. Teilchen nach Anspruch 16, wobei die funktionellen Gruppen monoklonale Antikörper sind.

19. Teilchen nach Anspruch 15, wobei die seitenständigen funktionellen Gruppen Aminogruppen sind.

20. Teilchen nach Anspruch 15, wobei die seitenständigen funktionellen Gruppen aus Maleimidylgruppen und Sulfhydrylgruppen ausgewählt sind.

21. Teilchen nach Anspruch 20, wobei eine biologische Substanz entweder an die Maleimidylgruppen oder die Sulfhydrylgruppen gebunden ist.

22. Teilchen nach Anspruch 21, wobei die biologische Substanz wie in einem der Ansprüche 16 bis 18 definiert ist.

23. Teilchen nach einem der Ansprüche 21 oder 22, wobei die biologische Substanz reaktive Substituenten umfasst, ausgewählt aus Sulfhydryl- und Maleimidylsubstituenten, mit der Maßgabe, daß, wenn die seitenständigen funktionellen Gruppen der Teilchen Maleimidylgruppen sind, die reaktiven Substituenten Sulfhydrylgruppen sind, und daß, wenn die seitenständigen funktionellen Gruppen Sulfhydrylgruppen sind, die reaktiven Substituenten Maleimidylgruppen sind.

24. Teilchen nach Anspruch 15, wobei die funktionellen Gruppen eine biologische Substanz umfassen, die an die äußerste Beschichtung des Kerns gebunden ist.

25. Teilchen nach Anspruch 24, wobei die biologische Substanz an die äußerste Beschichtung über eine bifunktionale Verbrückungsgruppe gebunden ist.

26. Teilchen nach Anspruch 25, wobei die Verbrückungsgruppe eine funktionelle Gruppe wie in den Ansprüchen 16 oder 17 definiert umfasst, bevorzugt ein Polyamin mit einer Amingruppe, gebunden an die äußerste Beschichtung, und eine andere Amingruppe oder Gruppen, gebunden an eine Einheit, welche eine reaktive Maleimidyl- oder Sulfhydrylgruppe aufweist, wobei das Polyamin aus Ethylendiamin, 1,3-Diaminopropan, 1,4-Cyclohexandiamin, 1,4-Cyclohexendiamin, 1,4-Phenylendiamin, Diethylentriamin und Aminodextranen ausgewählt ist.

27. Teilchen nach einem der Ansprüche 23 bis 26, wobei die Sulfhydrylgruppen auf der biologischen Substanz natürlich vorhanden sind oder durch Modifikation einer Aminogruppe oder Gruppen, die natürlich auf der biologischen Substanz vorhanden sind, unter Verwendung von 2-Iminothiolanhydrochlorid erzeugt worden sind, wobei die Maleimidylgruppen durch Modifikation einer Aminogruppe oder Gruppen auf der biologischen Substanz unter Verwendung eines Maleimidyl-enthaltenden Reagenzes erzeugt worden sind.

28. Verfahren zur Herstellung kollodialer Teilchen nach Anspruch 1, wobei das Verfahren umfasst:

(a) Beschichten des Kerns mit (i) einer alkaligehärteten Gelatine vom Typ B mit einer Gelstärke nach Bloom in dem Bereich von 60 bis 225; (ii) einer säuregehärteten Gelatine vom Typ A mit einer Gelstärke nach Bloom in dem Bereich von 60 bis 300 oder (iii) einem Aminodextran;
(b) Beschichten des Produkts aus den Schritten (a)(i) oder (a)(ii) mit einem Aminodextran;
(c) entweder (i) kein Vernetzen der Beschichtung des Produkts aus Schritt (a)(iii), wenn das Aminodextran kovalent an den Kern gebunden ist, oder (ii) Vernetzen der oder jeder Beschichtung des Produkts aus den Schritten (a)(iii) oder (b) mit einem chemischen Vernetzungsmittel;
(d) Blockieren jeglicher vorliegender freier, nicht reagierter, funktioneller Gruppen des Vernetzungsmittels durch Reaktion der funktionellen Gruppen mit einem Polyamin, wobei mindestens eine Aminogruppe des Polyamins unreagiert verbleibt, und
(e) Waschen des Produkts von Schritt (d) oder (c)(i).

29. Verfahren nach Anspruch 28, wobei das Produkt von Schritt (e) mit mindestens einer Verbindung umgesetzt wird, ausgewählt aus:

(i) einem Reagenz, welches in dem Produkt mit seitenständigen Sulfhydrylgruppen resultiert,

(ii) einem Reagenz, welches in dem Produkt mit seitenständigen Maleimidytgruppen resultiert, und
(iii) einem Reagenz, welches in dem Produkt mit seitenständigen biologischen Substanzen resultiert, vorzugsweise wie in einem der Ansprüche 16 bis 18 oder 23 definiert.

**30.** Verfahren nach Anspruch 28 oder 29, wobei das Polyamin wie nach Anspruch 26 definiert ist.

**31.** Verfahren nach einem der Ansprüche 28 bis 30, wobei in Schritt (a), wenn die Kernsubstanz eine magnetische Substanz ist, die Kernsubstanz mit einer Gelatine vom Typ B beschichtet wird, und wenn die Kernsubstanz eine Polymerverbindung ohne aminreaktive funktionelle Oberflächengruppen ist, die Kernsubstanz mit einer Gelatine vom Typ A beschichtet wird, und wenn die Kernsubstanz eine Polymersubstanz mit aminreaktiven funktionellen Gruppen ist, die Kernsubstanz mit einem Aminodextran beschichtet wird, und in Schritt (c)(i) das Aminodextran kovalent an die aminreaktiven Gruppen auf der Kemoberfläche gebunden wird.

**32.** Verfahren nach einem der Ansprüche 28 bis 31, wobei der Kern metallisch ist und das Verfahren in Schritt (a)

(i) das Herstellen metallischer Kernteilchen in einer Gelatine, bevorzugt in einer Gelatine vom Typ B, oder
(ii) das Adsorbieren einer Gelatine als erste Beschichtung an vorgeformte metallische Teilchen und das Adsorbieren eines Aminodextrans auf der Oberfläche der Gelatine-beschichteten Teilchen als zweite Beschichtung oder
(iii) das Adsorbieren einer Gelatine als erste Beschichtung auf vorgeformte metallische Teilchen und das Anbinden eines Aminodextrans an die Gelatine als zweite Beschichtung mittels einer Kondensationsreaktion umfasst,

wobei das Verfahren weiter nach Waschen gegebenenfalls das Derivatisieren der resultierenden Teilchen durch Umsetzung mit einem bifunktionalen Verbrückungsreagenz zum Einbringen seitenständiger funktioneller Gruppen umfasst, wobei die funktionellen Gruppen bevorzugt wie nach Anspruch 29 definiert sind.

**33.** Verfahren nach Anspruch 32, wobei die resultierenden Teilchen mit einem bifunktionalen Verbrückungsmittel umgesetzt werden.

**34.** Verfahren nach Anspruch 33, wobei die Gelatine eine Gelatine vom Typ B ist und worin (iii) des Anspruchs 32 durch Mischen des Kernmaterials mit einer 1%igen (GewichtNolumen) wässrigen Gelatinelösung durchgeführt wird, wobei die Umsetzung mit dem bifunktionalen Verbrückungsreagenz in einer phosphatgepufferten Kochsalzlösung bei Umgebungstemperatur für 0,5 bis 1,5 Stunden durchgeführt wird, um Teilchen mit reaktiven terminalen Maleimidyl- oder Sulfhydrylgruppen, gebunden an die Teilchenoberfläche, herzustellen, wobei das Verfahren weiter die folgenden Schritte umfasst:

(i) Herstellen eines Antikörpers zur Bindung an die Teilchen durch Erzeugung reaktiver Substituenten, welche aus Sulfhydrylgruppen und Maleimidylgruppen ausgewählt sind, auf dem Antikörper;
(ii) Umsetzen der Teilchen und des Antikörpers durch Mischen für 1 bis 3 Stunden, wobei die reaktiven Substituenten auf dem Antikörper an die funktionellen Gruppen auf den Teilchen gebunden werden, und Abtrennen der resultierenden Antikörper-enthaltenden Teilchen von dem Reaktionsmedium und Waschen mit gepufferter Kochsalzlösung;
(iii) Blockieren nicht reagierter Gruppen, die auf dem Produkt des vorstehenden Schrittes (ii) vorhanden sind, und
(iv) Abtrennen und Waschen der Antikörper-enthaltenden Teilchen mit einer Lösung von etwa 1% Bovine Serum Albumin in 0,1%igem Natriumazid in phosphatgepufferter Kochsalzlösung, Lagern der gewaschenen Teilchen in der Lösung bei etwa 4°C für 8 bis 16 Stunden, erneutes Waschen der Teilchen und Lagern bis zur benötigten Verwendung.

**35.** Verfahren nach Anspruch 34, wobei (ii) des Anspruchs 32 durch Waschen der Gelatine-beschichteten Teilchen mit einer Lösung eines Aminodextrans, Lagern der Teilchen für bis zu 6 Monate vor Suspendieren der Teilchen in einer Aminodextran-Beschichtungslösung durchgeführt wird, wobei das Verfahren vor der Umsetzung der Teilchen mit dem bifunktionalen Verbrückungsreagenz weiter die folgenden Schritte umfasst:

(i) Mischen der Teilchensuspension mit einer Lösung von Glutaraldehyd für etwa eine Stunde, wobei die Gelatine/Aminodextran-Oberfläche vernetzt wird;
(ii) Zugeben von Ethylendiamin zu der Suspension und Mischen für 1 bis 4 Stunden,

(iii) Zugeben von Natriumborhydrid zu der Suspension und Mischen,

(iv) Abtrennen der Teilchen von der Suspension und Waschen mit einer 0,2 M wässrigen NaCl;

(v) Umsetzen der Teilchen vom vorstehenden Schritt (iii) oder (iv) mit Ethylendiamin in einer 0,2 M wässrigen NaCl-Lösung, die 1-Ethyl-3-(3-diethylaminopropyl)carbodiimid enthält, unter Mischen bei Umgebungstemperatur und

(vi) Abtrennen der Teilchen und Waschen mit phosphatgepufferter Kochsalzlösung.

**36.** Verfahren nach einem der Ansprüche 28 bis 31, wobei der Kem eine polymere Substanz ist, die aminreaktive funktionelle Gruppen aufweist oder nicht, wobei das Verfahren weiter umfasst:

(A) Beschichten einer Gelatine, bevorzugt einer Gelatine vom Typ A, oder eines Aminodextrans auf die Kernoberfläche entweder durch:

(i) Adsorbieren der Gelatine oder des Aminodextrans auf der Kernoberfläche oder

(ii) kovalentes Anbinden des Aminodextrans an den Kern durch Umsetzung der Aminodextran-Amingruppen mit den aminreaktiven Gruppen auf dem Kern;

(B) (i) Vernetzen der Gelatine- oder Aminodextranbeschichtung des Schritts (A)(i) mittels eines chemischen Vernetzungsmittels oder (ii) Nichtvernetzen oder selektives Vernetzen der Aminodextranbeschichtung aus Schritt (A)(ii), wenn vorhanden, durch ein chemisches Vernetzungsmittel.

**Revendications**

**1.** Particules colloïdales distinctes ayant un enrobage de surface ayant un certain nombre de groupes fonctionnels pendants, les particules comprenant:

(a) un coeur solide, non-gélatine, non-aminodextrane

(b) un premier enrobage d'au moins une substance sélectionnée parmi

(i) une gélatine du type B, durcie aux alcalis de Bloom dans la gamme de 60 à 225 et

(ii) une gélatine du type A, durcie aux acides de Bloom dans la gamme de 60 à 300, et

(iii) un aminodextrane;

(c) quand l'enrobage est en gélatine du type A ou du type B, un second enrobage d'aminodextrane; et

(d) des groupes fonctionnels pendants attachés de manière covalente à l 'enrobage le plus externe; et

où le ou chaque enrobage du coeur est soit :

(I) réticulé par l'action d'un agent réticulant chimique, ou

(II) non réticulé ou réticulé par un agent réticulant chimique quand l'enrobage le plus externe est un aminodextrane qui est couplé de manière covalente au coeur.

**2.** Particules selon la revendication 1, où le coeur est sélectionné parmi

(i) uniquement une substance magnétique,

(ii) une substance polymérique, soit magnétique ou non-magnétique, qui a des groupes amino-réactifs de surface, et

(iii) une substance polymérique, soit magnétique ou non-magnétique, qui n'a pas de groupes fonctionnels amino-réactifs de surface;

et où le ou chaque enrobage aur le cour est soit:

(I) réticulé par l'action d'un agent réticulant chimique quand le coeur n'a pas de groupes amino-réactifs de surface, ou

(II) non réticulé ou réticulé par un agent réticulant chimique quand le premier enrobage est un aminodextrane et que le coeur a des groupes amino-réactifs de surface, l'aminodextrane étant couplé de manière covalente à la surface du coeur par réaction entre les groupes amino-réactifs et les groupes aminés sur l'aminodextrane.

3. Particules selon la revendication 1 ou la revendication 2, où le coeur est une substance polymérique et seul le premier enrobage est présent et est un aminodextrane.

4. Particules selon la revendication 2, où le coeur est sélectionné parmi une substance polymère seule ou une substance magnétique polymérique et le premier enrobage est un aminodextrane.

5. Particules selon la revendication 1 ou la revendication 2, où le premier enrobage comprend une gélatine du type B et le second enrobage comprend un aminodextrane, soit les deux couches d'enrobage étant réticulées sur la surface du coeur ou bien le premier enrobage étant adsorbé sur le substrat avec le second enrobage qui lui est joint par une réaction de condensation.

6. Particules selon toute revendication précédente, où le coeur comprend une substance magnétique.

7. particules selon la revendication 6, où la surface du coeur est hydrophobe.

8. Particules selon toute revendication précédente, où le coeur est dans la gamme de dimension de 0,1 à 5,0 microns.

9. Particules selon la revendication 8, où le coeur est dans la gamme de dimension de 0,1 à 1,0 micron.

10. Particules selon toute revendication précédente, où le caeur comprend du latex de polystyrène.

11. Particules selon toute revendication précédente où la surface du coeur a des groupes fonctionnels amino-réactifs sélectionnés dans le groupe consistant en aldéhyde, acides carboxyliques et esters, et groupes tosyles.

12. Particules selon toute revendication précédente où l'agent réticulant est du glutaraldéhyde.

13. Particules selon toute revendication précédente, où l'aminodextrane est sélectionné parmi 1X-, 2X-, 3X- et 5X-aminodextrane.

14. Particules selon la revendication 13, où l'aminodextrane est 5X-aminodextrane.

15. Particules selon toute revendication précédente, où les groupes fonctionnels pendants de l'enrobage le plus externe sont sélectionnés parmi des groupes amino, des groupes maléimidyles, des groupes sulfhydryles, et une substance biologique.

16. Particules selon la revendication 15, où les groupes fonctionnels pendants sont des substances biologiques sélectionnées parmi des anticorps monoclonaux, des anticorps polyclonaux et des cellules biologiques.

17. Particules selon la revendication 16, où les groupes fonctionnels sont des anticorps polyclonaux.

18. Particule selon la revendication 16, où les groupes fonctionnels sont des anticorps monoclonaux.

19. Particules selon la revendication 15, où les groupes fonctionnels pendants sont des groupes amino.

20. Particules selon la revendication 15, où les groupes fonctionnels pendants sont sélectionnés parmi les groupes maléimidyles et les groupes sulfhydryles.

21. Particules selon la revendication 20, où une substance biologique est liée aux groupes maléimidyles ou aux groupes sulfhydryles.

22. Particules selon la revendication 21, où la substance biologique est telle que définie dans l'une quelconque des revendications 16 à 18.

23. Particules selon l'une quelconque des revendications 21 ou 22, où la substance biologique comprend des substituants réactifs sélectionnés parmi des substituants sulfhydryles et des substituants maléimidyles, à condition que quand les groupes fonctionnels pendants de la particule sont des groupes maléimidyles, les substituants réactifs soient des groupes sulfhydryles, et quand les groupes fonctionnels pendants de la particule sont des groupes sulfhydryles, les substituants réactifs soit des groupes maléimidyles.

**24.** Particules selon la revendication 15, où les groupes fonctionnels comprennent une substance biologique qui est liée à l'enrobage le plus externe du coeur.

**25.** Particules selon la revendication 24, où la substance biologique est liée à l'enrobage le plus externe par un groupe pontant bifonctionnel.

**26.** Particules selon la revendication 25, où le groupe pontant comprend un groupe fonctionnel tel que défini à la revendication 16 ou à la revendication 17, étant de préférence une polyamine ayant un groupe amine lié à l'enrobage le plus externe et un autre groupe amine ou des groupes liés à une fraction ayant un groupe maléimidyle ou sulfhydryle réactif, la polyamine étant sélectionnée parmi éthylènediamine, 1,3-diaminopropane, 1,4-cyclohexanediamine, 1,4-cyclohexènediamine, 1,4-phénylènediamine, diéthylène triamine et aminodextranes.

**27.** Particules selon l'une quelconque des revendications 23 à 26, où les groupes sulfhydryles sont naturellement présents sur la substance biologique ou sont générés par modification d'un groupe amino ou deux groupes naturellement présents sur la substance biologique en utilisant du chlorhydrate de 2-iminothiolane, et les groupes maléimidyles sont générés par modification d'un groupe amino ou de groupes sur la substance biologique en utilisant un réactif contenant du maléimidyle.

**28.** Procédé de préparation de particules colloïdales telles que définies à la revendication 1, le procédé comprenant:

(a) l'enrobage du coeur avec

(i) une gélatine durcie aux alcalis du type B de Bloom dans la gamme de 60 à 225;
(ii) une gélatine durcie aux acides du type A, de Bloom dans la gamme de 60 à 300; ou
(iii) un aminodextrane;

(b) l'enrobage du produit des étapes (a) (i) ou (a) (ii) par un aminodextrane.
(c) soit (i) la non réticulation de l'enrobage du produit de l'étape (a)(iii) quand l'aminodextrane est couplé de manière covalente au coeur, ou (ii) la réticulation du ou de chaque enrobage des produits des étapes (a)(iii) ou (b) avec un agent réticulant chimique.
(d) le blocage de tous les groupes fonctionnels libres, présents, de l'agent réticulant n'ayant pas réagi par réaction desdits groupes avec une polyamine, au moins un groupe amino de la polyamine restant sans réagir; et
(e) le lavage du produit de l'étape (d) ou de l'étape (c) (i).

**29.** Procédé selon la revendication 28 où le produit de l'étape (e) est mis à réagir avec au moins une substance sélectionnée parmi:

(i) un réactif qui aura pour résultat le produit ayant des groupes sulfhydryles pendants
(ii) un réactif qui aura pour résultat le produit ayant des groupes maléimidyles pendants, et
(iii)un réactif qui aura pour résultat le produit ayant des substances biologiques pendantes, de préférence comme défini selon l'une quelconque des revendications 16 à la ou la revendication 23.

**30.** Procédé selon la revendication 28 ou la revendication 29, où la polyamine est telle que définie à la revendication 26.

**31.** Procédé selon l'une quelconque des revendications 28 à 30, où à l'étape (a) quand la substance du coeur est une substance magnétique, elle est enrobée d'une gélatine du type B; quand la substance du coeur est une substance polymérique n'ayant pas de groupes fonctionnels amino réactifs de surface, elle est enrobée d'une gélatine du type A; et quand la substance du coeur est une substance polymérique ayant des groupes fonctionnels aminoréactifs, elle est enrobée d'un aminodextrane, et à l'étape (c)(i) l'aminodextrane est couplé de manière covalente aux groupes amino-réactifs sur la surface du coeur.

**32.** Procédé selon l'une quelconque des revendications 28 à 31, où le coeur est métallique, le procédé comprenant dans l'étape (a):

(i) la préparation des particules de coeur métalliques dans une gélatine, de préférence une gélatine du type B; ou
(ii) l'adsorbtion, en tant que premier enrobage, d'une gélatine sur les particules métalliques préformées et l'adsorption, en tant que second enrobage, d'un aminodextrane sur la surface des particules enrobées de gélatine; ou

(iii) l'adsorbtion, en tant que premier enrobage, d'une gélatine sur les particules métalliques préformées et la jonction à la gélatine, par une réaction de condensation, d'un aminodextrane en tant que second enrobage,

le procédé comprenant de plus facultativement, après lavage, la dérivatisation des particules résultantes par réaction avec un réactif pontant bifonctionnel pour incorporer les groupes fonctionnels pendants, les groupes fonctionnels étant de préférence tels que définis à la revendication 29.

**33.** Procédé selon la revendication 32 où les particules résultantes sont mises à réagir avec un agent pontant bifonctionnel.

**34.** Procédé selon la revendication 33, où la gélatine est une gélatine du type B et où (iii) de la revendication 32 est effectuée, par mélange du matériau du coeur avec une solution aqueuse à 1% p/v de gélatine et où la réaction avec le réactif pontant bifonctionnel est effectuée dans une solution saline tamponnée de phosphate à température ambiante pendant 0,5 à 1,5 heures pour préparer des particules ayant des groupes maléimidyles ou sulfhydryles terminaux réactifs liés à la surface des particules, le procédé comprenant de plus les étapes suivantes:

(i) la préparation d'un anticorps pour liaison aux particules en générant des substituants réactifs sur cet anticorps qui sont sélectionnés parmi des groupes sulfhydryles et des groupes maléimidyles;
(ii) la réaction des particules et de l'anticorps, tout en mélangeant pendant 1 à 3 heures, pour ainsi coupler les substituants réactifs sur l'anticorps aux groupes fonctionnels sur les particules et séparer les particules contenant un anticorps résultantes du milieu réactionnel et laver avec une solution saline tamponnée;
(iii) bloquer les groupes n'ayant pas réagi présents sur le produit de l'étape (ii) ci-dessus; et
(iv) séparer et laver les particules contenant un anticorps avec à peu près 1% d'albumine de sérum bovin dans 0,1% de NaN$_3$ dans une solution saline tamponnée de phosphate, stocker les particules lavées dans cette solution aux environs de 4°C pendant 8 à 16 heures, relaver les particules et stocker jusqu'à ce qu'elles soient requises pour une utilisation.

**35.** Procédé selon la revendication 34 où (ii) de la revendication 32 est effectuée par lavage des particules enrobées de gélatine avec une solution d'un aminodextrane, stockage des particules pendant jusqu'à environ six mois avant mise en suspension des particules dans une solution d'enrobage d'aminodextrane, le procédé comprenant de plus, avant réaction des particules avec le réactif pontant bifonctionnel, les étapes suivantes:

(i) mélange de la suspension des particules avec une solution de glutaraldéhyde pendant environ 1 heure, pour ainsi réticuler la surface gélatine/aminodextrane;
(ii) ajouter de l'éthylènediamine à la suspension et mélager pendant 1 à 4 heures;
(iii) ajouter NaBH$_4$ à la suspension et mélanger;
(iv) séparer les particules de la suspension et laver avec NaCl aqueux 0,2 M;
(v) faire réagir, tout en mélangeant, les particules de l'étape (iii) ou (iv) ci-dessus avec de l'élhylènediamine dans une solution aqueuse de NaCl 0.2 M contenant 1-éthyl-3-(3-diéthylaminopropyl) carbodiimide à température ambiante; et
(vi) séparer les particules et les laver avec une solution saline tamponnée de phosphate.

**36.** Procédé selon l'une quelconque des revendications 28 à 31 où le coeur est une substance polymérique ayant ou non des groupes fonctionnels amino-réactifs, le procédé comprenant de plus:

(A) l'enrobage d'une gélatine, de préférence d'une gélatine du type A, ou d'un aminodextrane sur la surface du coeur soit par:

(i) adsorbtion de la gélatine ou de l' aminodextrane sur la surface du coeur ou
(ii) couplage covalent de l'aminodextrane au coeur par réaction des groupes aminé de l'aminodextrane avec les groupes amino-réactifs sur le coeur;

(B) (i) la réticulation de l'enrobage de gélatine ou d'aminodextrane de l'étape (A) (i) en utilisant un agent réticulant chimique ou (ii) la non réticulation ou la réticulation sélective avec un agent réticulant chimique de l'enrobage d'aminodextrane de l'étape (A) (ii), quand il est présent.

# Fig. 1

EP 0 665 955 B1

# Fig. 2A

FS

LSS

# Fig. 2B

FS

LSS

# Fig. 2C

FS

LSS

# Fig. 2D

FS

LSS

# Fig. 2E

FS

LSS

# Fig. 2F

FS

LSS

# Fig. 2G

FS

LSS

# Fig. 2H

FS

LSS

# Fig. 2I

FS

LSS

# Fig. 2J

FS

LSS

# Fig. 2K

FS

LSS

# Fig. 2L

FS

LSS

46

# Fig. 3

# Fig. 4

# Fig. 5

# Fig. 6